# EUROPEAN PATENT SPECIFICATION

(11) **EP 3 977 964 B1**
(45) Date of publication and mention of the grant of the patent: **24.09.2025**
(21) Application number: 21199090.8
(22) Date of filing: 27.09.2021
(51) Int. Cl.: A61F 2/32

(54) **MAGNETICALLY STABILIZED TOTAL HIP REPLACEMENT PROSTHESIS**
MAGNETISCH STABILISIERTE HÜFTGELENKTOTALPROTHESE
PROTHÈSE DE HANCHE TOTALE MAGNÉTIQUEMENT STABILISÉE

(30) Priority: 01.10.2020 US 202017060993
(43) Date of publication of application: 06.04.2022
(73) Proprietor: Fellowship of Orthopaedic Researchers, Inc., Metairie LA 70005 (US)
(72) Inventor: Cook, Stephen D., Metairie, 70005 (US); Nolan, Liam P., Metairie, 70005 (US)
(74) Representative: Goddar, Heinz J.

(56) References cited:
- WO-A2-2008/057565
- US-A1- 2003 236 572
- US-A1- 2010 145 464
- US-A1- 2018 014 838

## Description

### FIELD OF INVENTION

The invention is associated with the field of orthopaedic implants, in particular total hip replacement (THR) prostheses and their use. The closest prior art is document US 2003/236572 A1, which defines the preamble of claim 1.

### BACKGROUND OF THE INVENTION

The hip joint-the joint between the femur and the acetabulum of the pelvis-is very durable, capable of tolerating high functional loads, large ranges in hip motion, and millions of cycles of repetitive use over a lifetime. Scientifically referred to as the acetabulofemoral joint, the hip joint has the capacity to repair and recover from activities of daily use. However, due to damage, disease, or injury, the motion of the hip joint can become painful and limited. As an example, osteoarthritis, a disease that affects the articulating joints of the body by causing breakdown of the cartilage that cover the bone surfaces of the joint, can result in pain and restricted movement.

As a treatment for a damaged or diseased hip joint, total hip replacement (THR) is one of the most successful surgical procedures in the field of orthopaedics. For patients with osteoarthritis of the hip, it offers significant pain relief, improved quality of life, and increased mobility in both the medium and long term. The prosthesis used for THR is similar to the natural ball-and-socket anatomy of the hip joint. The prosthesis comprises an acetabular component and a femoral component. The acetabular component typically has a full or partial hemispherical shape, with a liner that most often is fabricated from ultra-high molecular weight polyethylene. The femoral component generally features a metallic stem with a neck that attaches to a metallic or ceramic ball (i.e., femoral component head) that fits into the acetabular component. The articulation of the metal or ceramic ball with the polyethylene liner provides for low friction joint movement. Stability of the hip joint is achieved by the ball-and-socket design and the soft tissues and musculature surrounding the hip joint.

Dislocation of the femoral component from the acetabular component can be a devastating complication that can seriously affect a patient's quality of life. The prevalence of dislocation after primary THR (i.e., the first hip replacement surgery) has been reported to range from 0.2% to 7%, while the prevalence of dislocation after revision THR (i.e., subsequent hip replacement surgery that removes some or all of the parts of the original prosthesis) may range from 10% to as high as 28%. Approximately 2% of all patients dislocate their THR within one year of surgery, with most occurring in the first six to eight weeks when the soft tissues are healing. Reoperation for a dislocation is known to carry the highest likelihood of failure of any reoperation after THR, with a redislocation rate of 20% to 40%. Risk factors for dislocation include both patient-based factors such as neuromuscular and cognitive disorders, non-compliance with postoperative instructions, advanced age, and the female gender; and surgical-based factors such as the approach, soft tissue tensioning, femoral and acetabular component positioning, impingement, head size, acetabular liner profile, and surgeon experience.

Various surgical techniques and prosthesis design features have been developed to reduce the incidence of THR dislocation, but these solutions have their own complications and limitations. For example, improvements in posterior soft tissue repair during THR can increase stability around the hip joint, but such repair is often not possible due to the condition of the soft tissue and it requires a longer surgery.

The use of acetabular liners with a posteriorly oriented rim can provide greater capture of the femoral head within the acetabular cup, but it is accompanied by an increased risk of impingement due to contact occurring between the neck of the femoral component stem and the acetabular component, as well as liner wear, osteolysis, and loosening.

In addition, the use of larger femoral component heads have theoretical advantages in regard to stability and reducing dislocation, as the improved head to neck ratio can reduce impingement and the larger size indicates that the femoral head can be positioned deeper within the acetabular liner and would require a greater translation "jump distance" before dislocation. However, the use of larger femoral component heads has historically been limited by the risk of increased liner wear leading to osteolysis and loosening, and concerns for the potential of adverse local tissue reaction secondary to increased corrosion at the junction between the femoral head and neck due to higher torsional forces created by the larger head size.

Further, the use of constrained femoral head-polyethylene liners has been reported to help restore stability and reduce dislocation in revision THR for recurrent dislocation. However, constrained liners result in restricted range of motion for the patient and have a greater prevalence of impingement of the femoral neck on the acetabular cup, which can lead to high stress transmission to multiple interfaces and lead to liner damage, locking mechanism failure, dislocation, and loosening.

Another solution that has been studied is the use of a dual mobility acetabular component, which positions a mobile polyethylene component between the prosthetic head and the highly polished inner surface of an outer metal acetabular shell, therefore producing two bearings. The dual mobility provides a greater effective head size and improved head-to-neck ratio leading to improved range of motion to impingement and dislocation. Yet, dual mobility components raise concerns regarding the potential for increased polyethylene wear or damage as well as corrosion issues at the femoral head-neck interface.

Thus, there remains a need in the art for a THR prosthesis that can reduce the incidence of, or prevent, hip dislocation.

### SUMMARY OF INVENTION

In one aspect, the present invention relates to a THR prosthesis as defined in claim 1.

In some embodiments, in the acetabular component, the convex inner surface of the shell is configured to articulate with the outer surface of the spherical head.

In some embodiments, the acetabular component further comprises a liner that comprises a concave inner surface, a convex outer surface, and a thickness between the concave inner surface and the convex outer surface. The concave inner surface of the shell is configured to receive all or a portion of the convex outer surface of the liner. In certain embodiments, the concave inner surface of the liner is configured to articulate with the outer surface of the spherical head.

In the acetabular component, the one or more magnets may be at or near the central dome of the acetabular component, at the periphery of the acetabular component, in the intermediate wall between the central dome and the periphery of the acetabular component, or a combination thereof. The one or more magnets comprise a single magnet at the central dome. The long axis of the single magnet at the central dome may be perpendicular to the tangent line of the curvature at the central dome. The one or more magnets may comprise an array of magnets at the central dome.

According to the invention, in the acetabular component, the one or more magnets comprise a single magnet at the central dome and an array of magnets surrounding the single magnet at the central dome. The magnets in the array surrounding the single magnet at the central dome are equidistant from the single magnet at the central dome and may be equidistant from each other. In certain embodiments, the array comprises 2 to 16 magnets, or comprises 4 to 12 magnets.

The single magnet at the central dome may be oriented such that the long axis of the single magnet is perpendicular to the tangent line of the curvature at the central dome. In certain embodiments, the magnets surrounding the single magnet at the central dome are oriented such that the long axis of the surrounding magnets is parallel to the long axis of the central magnet. Or, in certain embodiments, the magnets surrounding the single magnet at the central dome are oriented such that the long axis of the surrounding magnets is angled to the long axis of the central magnet. The angle between the long axis of the magnets surrounding the single magnet at the central dome and the long axis of the single magnet may be about 10° to about 80°, or may be about 30° to about 60°.

In embodiments of the invention, in the femoral component, the tapered volume extends inward from the outside surface of the spherical head. The tapered volume may be defined by an end surface that is the innermost surface of the tapered volume, and walls that extend from the outside surface to the end surface. In certain embodiments, the cross-sectional area of the tapered volume decreases towards the end surface from the outside surface of the spherical head.

In some embodiments, in the femoral component, the one or more magnets are at the end surface of the tapered volume, at the walls of the tapered volume, embedded into the thickness of the spherical head adjacent to the end surface of the tapered volume, embedded into the thickness of the spherical head adjacent to the walls of the tapered volume, or a combination thereof. In certain embodiments, the one or more magnets comprise a single magnet at the end surface. The single magnet at the end surface may be oriented such that the long axis of the single magnet is perpendicular to the end surface. Alternatively, or in combination, the single magnet at the end surface may be oriented such that the long axis of the single magnet in the femoral component is parallel to the single magnet in the acetabular component when the total hip replacement prosthesis is in a 0° hip position.

In some embodiments, in the femoral component, the one or more magnets comprise an array of magnets at the end surface.

In some embodiments, in the femoral component, the one or more magnets comprise a single magnet at the end surface and an array of magnets surrounding the single magnet at the end surface. The magnets in the array surrounding the single magnet at the end surface may be equidistant from the single magnet at the end surface, are equidistant from each other, or a combination thereof. The array may comprise 2 to 16 magnets, or 4 to 12 magnets.

In certain embodiments, in the femoral component, the magnets surrounding the single magnet at the end surface are oriented such that the long axis of the surrounding magnets is parallel to the long axis of the central magnet.

In other embodiments, in the femoral component, the magnets surrounding the single magnet at the end surface are oriented such that the long axis of the surrounding magnets is angled to the long axis of the central magnet. The angle between the long axis of the magnets surrounding the single magnet at the end surface and the long axis of the single magnet may be about 10° to about 80°, or about 30° to about 60°.

In embodiments of the invention, the one or more magnets of the acetabular component and/or the one or more magnets of the femoral component are in their respective components by press fit, screw design, or taper lock. The magnets may comprise a cylindrical, disc, prism, conal, or pyramidal shape.

In embodiments in which the magnets are cylindrical, the one or more magnets in the acetabular component may comprise a diameter of about 2 mm to about 20 mm, or about 4 mm to about 15 mm; and a length of about 1 mm to about 15 mm, or about 3 mm to about 10 mm. The one or more magnets in the femoral component may comprise a diameter of about 1 mm to about 30 mm, or about 5 mm to about 20 mm; and a length of about 2 mm to about 30 mm, or about 5 mm to about 20 mm.

In some embodiments, the magnets comprise a magnetic material of a rare-earth magnet. For example, the magnetic material may be an alloy of neodymium, iron, and boron. In certain embodiments, the magnets are magnetized along the long axis of the magnets, or in a radial orientation.

In some embodiments, the magnets comprise a casing that encloses the magnetic material.

Methods are disclosed involving the use of the THR prosthesis of the invention. Some examples relate to a method of treating a subject in need of a THR. Some examples relate to a method of stabilizing a THR in a subject. Some examples relate to a method of reducing incidence of THR dislocation in a subject. Some examples relate to a method of reducing risk of THR dislocation in a subject. Some examples relate to a method of reducing risk of THR dislocation due to impingement in a subject. Some examples relate to a method of reducing risk of THR subluxation in a subject. And some examples relate to a method of reducing osteolysis associated with THR in a subject. These methods may comprise implanting the THR prosthesis of the present invention in the subject.

In one aspect, the present invention relates to a THR as defined in claim 15.

In some embodiments, in the acetabular component, the convex inner surface of the shell is configured to articulate with the outer surface of the spherical head.

In some embodiments, the acetabular component further comprises a liner that comprises a concave inner surface, a convex outer surface, and a thickness between the concave inner surface and the convex outer surface. The concave inner surface of the shell is configured to receive all or a portion of the convex outer surface of the liner. In certain embodiments, the concave inner surface of the liner is configured to articulate with the outer surface of the spherical head.

In the acetabular component, the one or more bores may be at or near the central dome of the acetabular component, at the periphery of the acetabular component, in the intermediate wall between the central dome and the periphery of the acetabular component, or a combination thereof. The one or more bores comprise a single bore at the central dome. The long axis of the single bore at the central dome may be perpendicular to the tangent line of the curvature at the central dome. The one or more bores comprise an array of bores at the central dome.

According to the invention, in the acetabular component, the one or more bores comprise a single bore at the central dome and an array of bores surrounding the single bore at the central dome. The bores in the array surrounding the single bore at the central dome are equidistant from the single bore at the central dome and may be equidistant from each other. In certain embodiments, the array comprises 2 to 16 bores, or comprises 4 to 12 bores.

The single bore at the central dome may be oriented such that the long axis of the single bore is perpendicular to the tangent line of the curvature at the central dome. In certain embodiments, the bores surrounding the single bore at the central dome are oriented such that the long axis of the surrounding bores is parallel to the long axis of the central bore. Or, in certain embodiments, the bores surrounding the single bore at the central dome are oriented such that the long axis of the surrounding bores is angled to the long axis of the central bore. The angle between the long axis of the bores surrounding the single bore at the central dome and the long axis of the single bore may be about 10° to about 80°, or may be about 30° to about 60°.

In embodiments of the invention, in the femoral component, the tapered volume extends inward from the outside surface of the spherical head. The tapered volume may be defined by an end surface that is the innermost surface of the tapered volume, and walls that extend from the outside surface to the end surface. In certain embodiments, the cross-sectional area of the tapered volume decreases towards the end surface from the outside surface of the spherical head.

In some embodiments, in the femoral component, the one or more bores are at the end surface of the tapered volume, at the walls of the tapered volume, embedded into the thickness of the spherical head adjacent to the end surface of the tapered volume, embedded into the thickness of the spherical head adjacent to the walls of the tapered volume, or a combination thereof. In certain embodiments, the one or more bores comprise a single bore at the end surface. The single bore at the end surface may be oriented such that the long axis of the single bore is perpendicular to the end surface. Alternatively, or in combination, the single bore at the end surface may be oriented such that the long axis of the single bore in the femoral component is parallel to the single bore in the acetabular component when the total hip replacement prosthesis is in a 0° hip position.

In some embodiments, in the femoral component, the one or more bores comprise an array of bores at the end surface.

In some embodiments, in the femoral component, the one or more bores comprise a single bore at the end surface and an array of bores surrounding the single bore at the end surface. The bores in the array surrounding the single bore at the end surface may be equidistant from the single bore at the end surface, are equidistant from each other, or a combination thereof. The array may comprise 2 to 16 bores, or 4 to 12 bores.

In certain embodiments, in the femoral component, the bores surrounding the single bore at the end surface are oriented such that the long axis of the surrounding bores is parallel to the long axis of the central bore.

In other embodiments, in the femoral component, the bores surrounding the single bore at the end surface are oriented such that the long axis of the surrounding bores is angled to the long axis of the central bore. The angle between the long axis of the bores surrounding the single bore at the end surface and the long axis of the single bore may be about 10° to about 80°, or about 30° to about 60°.

### BRIEF DESCRIPTION OF THE DRAWING FIGURES

The present disclosure will be further explained with reference to the attached drawing figures, wherein like structures are referred to by like numerals throughout the several views. The drawing figures shown are not necessarily to scale, with emphasis instead generally being placed upon illustrating the principles of the present disclosure, and some features may be exaggerated to show details of particular components. In addition, any measurements, specifications, and the like shown in the drawing figures, or described below, are intended to be illustrative, and not restrictive. Therefore, specific structural and functional details disclosed herein are not to be interpreted as limiting, but merely as a representative basis for teaching one skilled in the art to variously employ the magnetic devices and methods of their use.

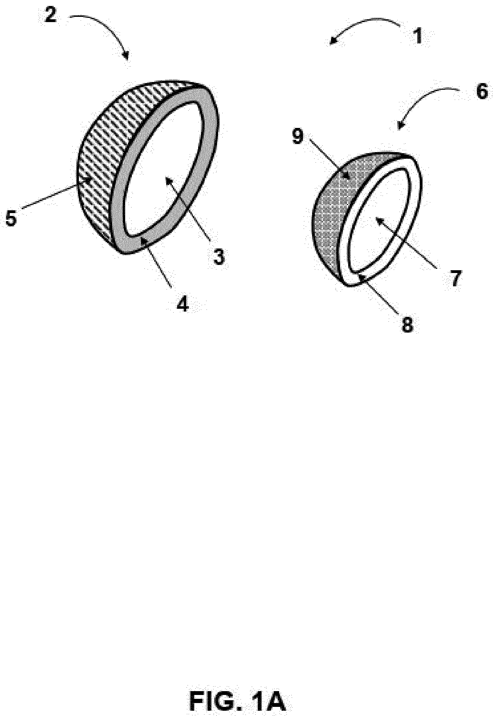

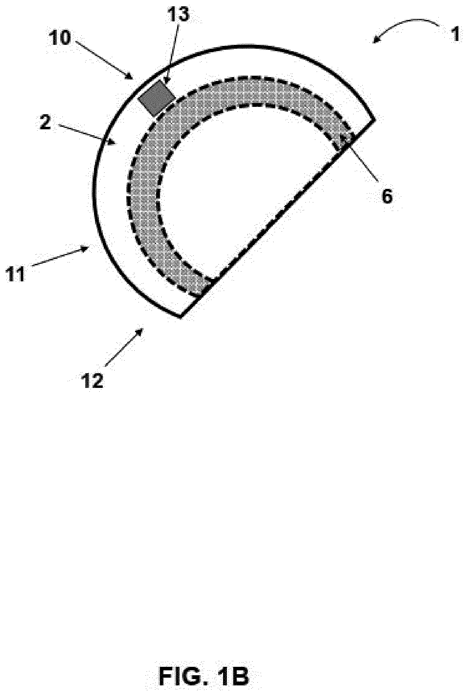

Figures 1A and 1B show the acetabular component of the THR prosthesis. Figure 1A shows an exploded view of the acetabular component, and Figure 1B shows a cut-away view of the acetabular component.

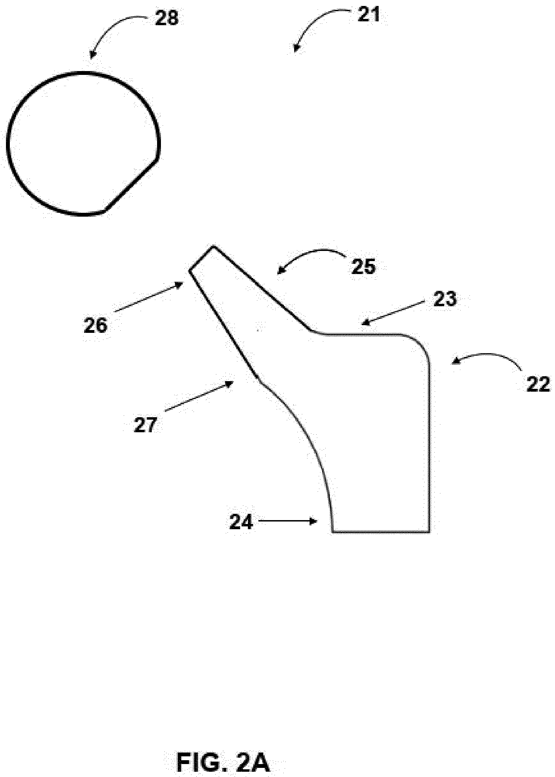

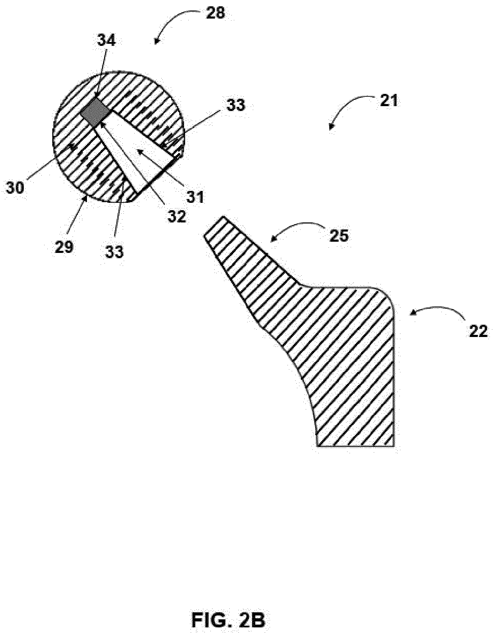

Figures 2A and 2B show the femoral component of the THR prosthesis, according to embodiments of the invention. Figure 2A shows an exploded perspective view of the femoral component, and Figure 2B shows an exploded cut-away view of the femoral component.

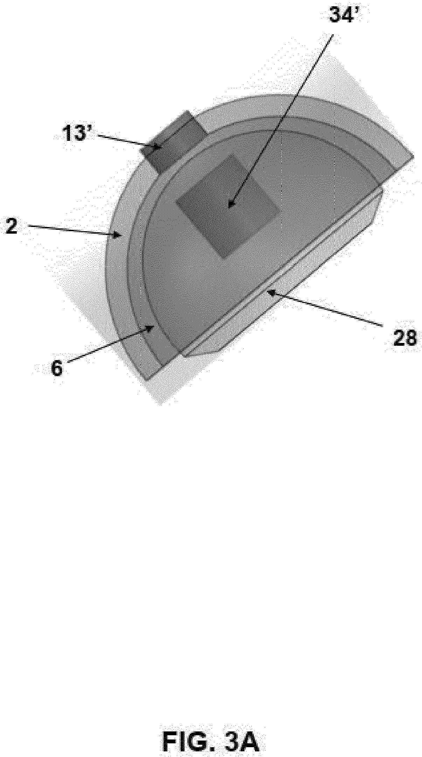

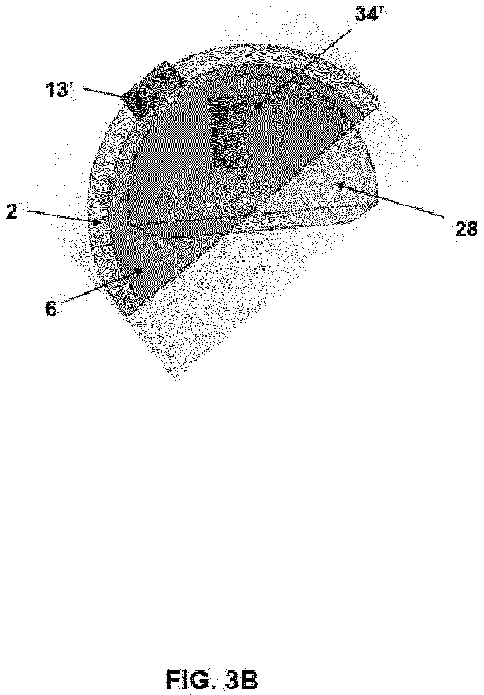

Figures 3A and 3B show the acetabular component and femoral component according to examples that were modeled in Example 1, in which the acetabular component comprised a single cylindrical magnet at the central dome of the shell, and the femoral component comprised a single cylindrical magnet at the end surface of the tapered volume in the spherical head. Figure 3A shows the positioning of the acetabular component and femoral component at the 0° hip position, and Figure 3B shows the positioning of the acetabular component and femoral component at the 35° hip position.

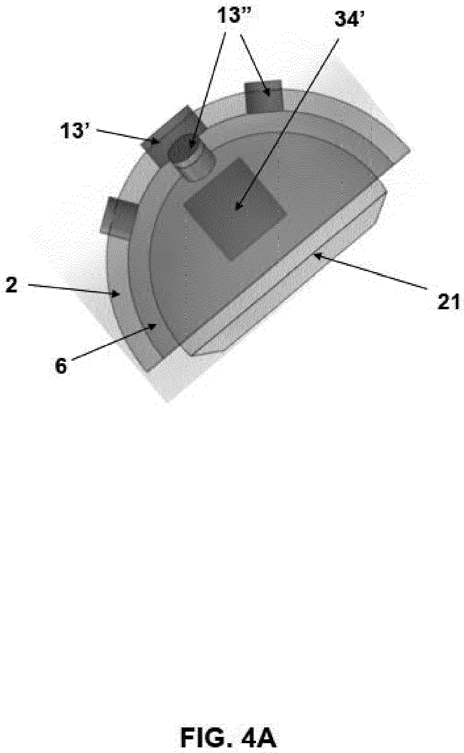

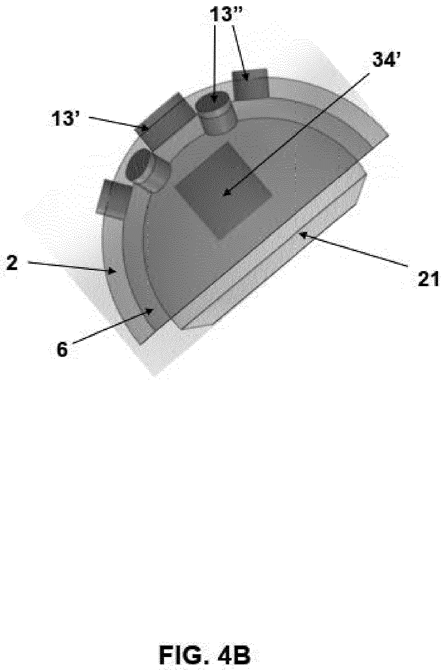

Figures 4A and 4B show the acetabular component and femoral component according to embodiments of the invention that were modeled in Example 2. The acetabular component comprised a single cylindrical magnet at the central dome surrounded by an array of magnets, in which the long axis of the magnets in the array was angled at 35° to the long axis of the central magnet. The femoral component comprised a single cylindrical magnet at the end surface of the tapered volume in the spherical head. Figure 4A shows the array in the acetabular component having four magnets, and Figure 4B shows the array in the acetabular component having six magnets.

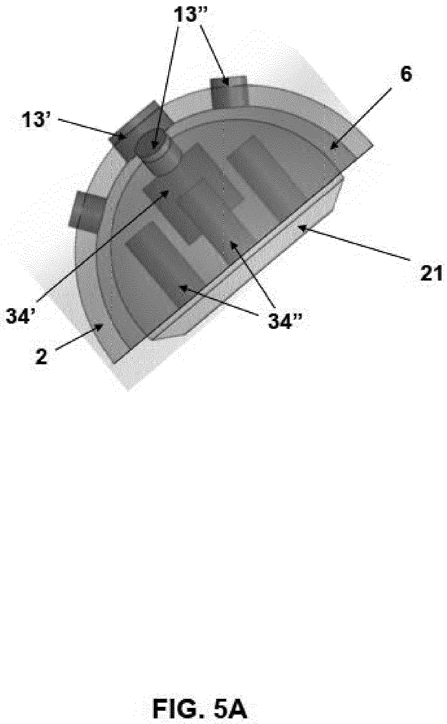

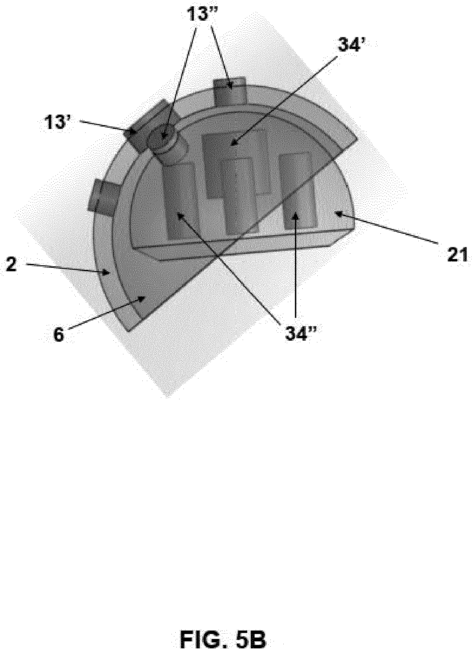

Figures 5A and 5B show the acetabular component and femoral component according to embodiments of the invention that were modeled in Example 3. The acetabular component comprised a single cylindrical magnet at the central dome surrounded by an array of magnets, in which the long axis of the magnets in the array was angled at 35° to the long axis of the central magnet. The femoral component comprised a single cylindrical magnet at the end surface of the tapered volume surrounded by an array of magnets in the spherical head, in which the long axis of the magnets in the array was parallel to the long axis of the magnet at the end surface. Figure 5A shows the positioning of the acetabular component and femoral component at the 0° hip position, and Figure 5B shows the positioning of the acetabular component and femoral component at the 35° hip position.

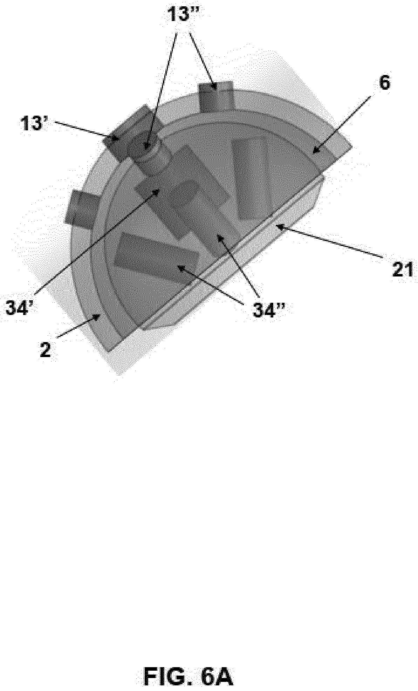

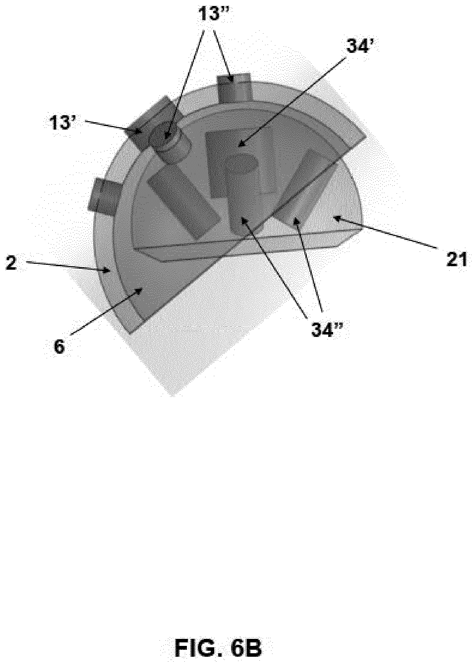

Figures 6A and 6B show the acetabular component and femoral component according to embodiments of the invention that were modeled in Example 4. The acetabular component comprised a single cylindrical magnet at the central dome surrounded by an array of magnets, in which the long axis of the magnets in the array was angled at 35° to the long axis of the single magnet at the central dome. The femoral component comprised a single cylindrical magnet at the end surface of the tapered volume surrounded by an array of magnets in the spherical head, in which the long axis of the magnets in the array was angled at 35° to the long axis of the central magnet at the end surface. Figure 6A shows the positioning of the acetabular component and femoral component at the 0° hip position, and Figure 6B shows the positioning of the acetabular component and femoral component at the 35° hip position.

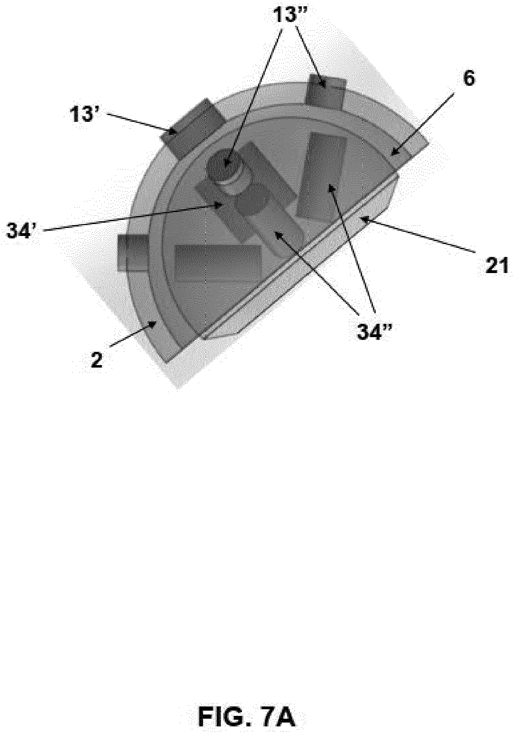

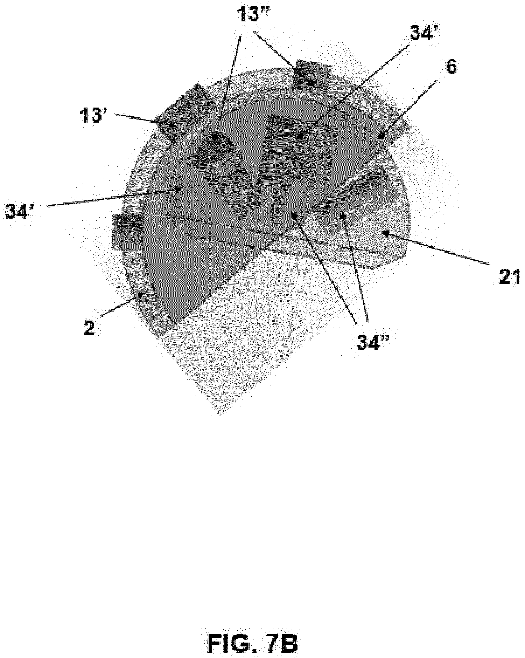

Figures 7A and 7B show the acetabular component and femoral component according to embodiments of the invention that were modeled in Example 4. The acetabular component comprised a single cylindrical magnet at the central dome surrounded by an array of magnets, in which the long axis of the magnets in the array was angled at 50° to the long axis of the single magnet at the central dome. The femoral component comprised a single cylindrical magnet at the end surface of the tapered volume surrounded by an array of magnets in the spherical head, in which the long axis of the magnets in the array was angled at 50° to the long axis of the central magnet at the end surface. Figure 7A shows the positioning of the acetabular component and femoral component at the 0° hip position, and Figure 7B shows the positioning of the acetabular component and femoral component at the 50° hip position.

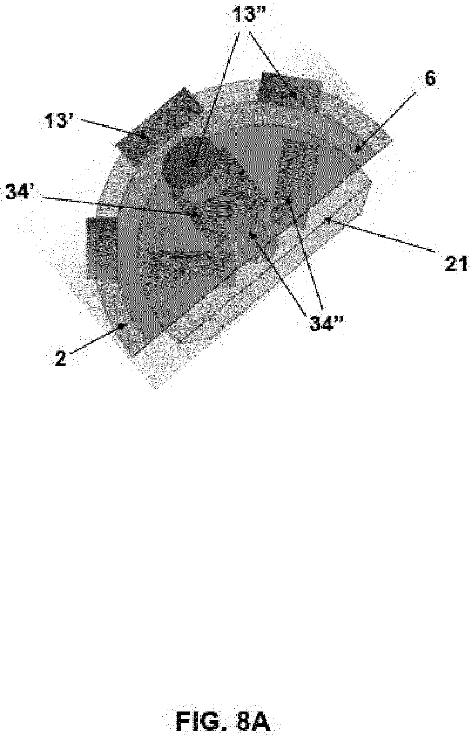

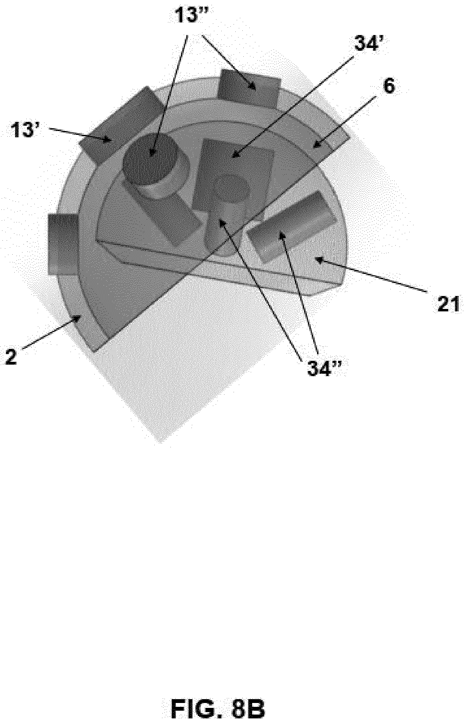

Figures 8A and 8B show the acetabular component and femoral component according to embodiments of the invention that were modeled in Example 5. The acetabular component comprised a single cylindrical magnet at the central dome surrounded by an array of magnets, in which the long axis of the magnets in the array was angled at 50° to the long axis of the single magnet at the central dome. The femoral component comprised a single cylindrical magnet at the end surface of the tapered volume surrounded by an array of magnets in the spherical head, in which the long axis of the magnets in the array was angled at 50° to the long axis of the central magnet at the end surface. Figure 8A shows the positioning of the acetabular component and femoral component at the 0° hip position, and Figure 8B shows the positioning of the acetabular component and femoral component at the 50° hip position.

### DETAILED DESCRIPTION OF THE INVENTION

Aspects of the present disclosure relate to a THR prosthesis, methods of using the THR prosthesis, methods of manufacturing the THR prosthesis, and kits for manufacturing the THR prosthesis.

The THR prosthesis comprises an acetabular component and a femoral component, in which each component comprises one or more embedded magnets. An attractive force is generated between the one or more magnets of the acetabular component and the one or more magnets of the femoral component, which provides greater stability between the components and reduces the risk of dislocation. The present invention avoids the complications and limitations of conventional methods of stabilizing THR prostheses that can reduce the range of motion, increase potential for wear and damage to components, and increase taper corrosion.

### THR Prosthesis

The THR prosthesis of the present invention comprises an acetabular component and a femoral component. Each component comprises one or more magnets.

### Acetabular Component

As demonstrated in Figures 1A and 1B, the acetabular component **1** of the THR prosthesis may comprise a hollowed, full or partial hemispherical shape. In some examples, the acetabular component **1** comprises a shell **2,** which comprises a concave inner surface **3,** a convex outer surface **5,** and a thickness **4** between the inner surface **3** and the outer surface **5.** In some examples, the acetabular component **1** further comprises a liner **6,** which comprises a concave inner surface **7,** a convex outer surface **9,** and a thickness **8** between the inner surface **7** and the outer surface **9.** The liner **6** is configured to fit within the shell **2,** such that the outer surface **9** of the liner **6** is adjacent to the inner surface **3** of the shell **2.** In some examples, the liner **6** may be a constrained liner.

The acetabular component **1** may comprise materials known in the art for acetabular components of THR prostheses. For instance, the acetabular component **1** may comprise a metal such as stainless steel, titanium, chromium, cobalt, or a combination thereof; a plastic such as polyethylene or cross-linked polyethylene; or a ceramic. Notably, the shell **2** and liner **6** of the acetabular component **1** may comprise different materials. For example, the shell **2** may comprise a metal while the liner **6** may comprise a plastic or ceramic.

The acetabular component **1** may comprise one or more magnets **13** positioned at or near the central dome **10** of the acetabular component **1,** at the periphery **12** of the acetabular component **1,** in the intermediate wall **11** between the central dome **10** and the periphery **12** of the acetabular component **1,** or a combination thereof. In certain examples, one of more magnets **13** are positioned at the central dome **10** of the acetabular component **1.** In certain examples, one of more magnets **13** are positioned at both the central dome **10** and in the intermediate wall **11** of the acetabular component **1.**

The number of magnets and their position in the acetabular component **1** may vary. For example, in some examples, a single magnet **13'** may be positioned at the central dome **10** of the acetabular component 1; an example of such an arrangement is demonstrated in Figures 1B, 3A, and 3B. An array of magnets **13"** may surround the central dome **10** of the acetabular component **1,** in which each magnet in the array is positioned at an approximately equal distance from the central dome **10** and/or is positioned at an approximately equal distance from each other (not shown). The array **13"** may comprise 2 to 16 magnets, or 4 to 12 magnets, or a number therebetween, such as 2 magnets, or 3 magnets, or 4 magnets, or 5 magnets, or 6 magnets, or 7 magnets, or 8 magnets, or 9 magnets, or 10 magnets, or 11 magnets, or 12 magnets, or 13 magnets, or 14 magnets, or 15 magnets, or 16 magnets.

According to the invention, magnets are positioned in the acetabular component **1** such that one magnet **13'** is at the central dome **10** (i.e., the "central magnet") and an array of magnets **13"** surround the central magnet and are positioned at an approximately equal distance from the central magnet **13'** and, optionally, positioned at an approximately equal distance from each other; an example of such an arrangement is demonstrated in Figures 4A through 8B. In certain embodiments, the magnets **13"** surrounding the central magnet **13'** may be positioned at the intermediate wall **11** of the acetabular component **1,** as illustrated in Figures 4A to 8B. In some embodiments, the magnets **13"** surrounding the central magnet **13'** may be positioned at the periphery **12** of the acetabular component **1** (not shown). In some embodiments, the array **13"** may comprise 2 to 16 magnets, or 4 to 12 magnets, or a number therebetween, such as 2 magnets, or 3 magnets, or 4 magnets, or 5 magnets, or 6 magnets, or 7 magnets, or 8 magnets, or 9 magnets, or 10 magnets, or 11 magnets, or 12 magnets, or 13 magnets, or 14 magnets, or 15 magnets, or 16 magnets.

In embodiments in which the acetabular component **1** includes a central magnet **13',** the central magnet **13'** may be oriented such that the long axis of the magnet is perpendicular to the tangent line of the curvature at the central dome. In some embodiments, the central magnet **13'** may be oriented such that the long axis of the magnet is perpendicular to the tangent line of the curvature of the inner surface **3** of the shell **2** at the central dome **10.** In certain embodiments in which the acetabular component **1** comprises a liner **6,** the central magnet **13'** may be oriented such that the long axis of the magnet is perpendicular to the tangent line of the curvature of the inner surface **7** of the liner **6** at the central dome **10.**

In embodiments in which an array of magnets **13"** surround a central magnet **13',** the long axis of the surrounding magnets **13"** may be parallel to the long axis of the central magnet **13'.** Alternatively, the long axis of the surrounding magnets **13"** may be angled to the long axis of the central magnet **13',** as demonstrated in Figures 4A to 8B. This angle may be about 10° to about 80°, or about 20° to about 70°, or about 30° to about 60°, or any angle therebetween, such as about 35°, or about or about 40°, or about or about 45°, or about 50°.

In some embodiments, the one or more magnets **13** in the acetabular component **1** may also be positioned such that the distance between the one or more magnets **13** and the inner surface **3** of the shell **2,** or the inner surface **7** of the liner **6** in embodiments in which a liner **6** is present, may be no more than about 5 mm, or no more than about 4 mm, or no more than about 3 mm, or no more than about 1 mm.

The one or more magnets **13** may be in the acetabular component **1** through means known in the art, including affixing by a press fit, screw designs (e.g., a screw with a magnet is screwed into the acetabular component), taper lock, etc. In some embodiments, the one or more magnets **13** are positioned in the acetabular component **1** via one of the surfaces of the shell **2** (e.g., the magnet(s) **13** are press-fitted, screwed into, locked in, etc., into the surface of the shell **1**), such as the outer surface **5,** the inner surface **3,** or a combination thereof. In some embodiments, the one or more magnets **13** are positioned in the acetabular component **1** via one of the surfaces of the liner **6** (e.g., the magnet(s) **13** are press-fitted, screwed into, locked in, etc., into the surface of the liner **6**), such as the outer surface **9,** the inner surface **7,** or a combination thereof. In some embodiments, the one or more magnets **13** may be embedded within the thickness **4** of the shell **2,** within the thickness **8** of the liner **6,** or a combination thereof. In certain embodiments, the one or more magnets **13** may be positioned in the acetabular component **1** by a combination of affixing to a surface of the shell **2** and/or liner **6,** and/or embedding within the thickness **4** of the shell **2** and/or within the thickness **8** of the liner **6.** In certain embodiments, one or more recesses may be cut into the shell **2** and/or the liner **6** so that individual magnet(s) may be positioned in both the shell **2** and the liner **6,** e.g., extends through the inner surface **3** of the shell **2** and through the outer surface **9** of the liner **6.,** but not through inner surface **7** of the liner **6.**

The magnet(s) may be generally any geometric shape, such as a cylinder, disc, prism (including rectangular prism, hexagonal prism, triangular prism, cube, etc.), cone, and pyramid. In certain embodiments, the one or more magnets are cylindrical. If more than one magnet is present, each magnet may comprise the same or a different geometric shape.

In embodiments in which the magnet is cylindrical or disc-shaped, the "long axis" refers to the line that is formed by the centers of the circular bases of the cylinder or disc. In embodiments in which the magnet is prism-shaped, the "long axis" refers to the line formed by the centers of bases in the direction of the longest dimension of the magnet. In embodiments in which the magnet is conal or pyramidal, the "long axis" refers to the line between the apex and the center of the base and perpendicular to the base.

In some embodiments, a surface of the magnet may comprise a geometric contour, such as a curvature, that is similar to the curvature of the shell and/or the curvature of the liner.

The magnet(s) may comprise magnetic materials known in the art. For example, the magnetic materials may be iron-based, nickel-based, cobalt-based, or an alloy of rare-earth metals. In some embodiments, the magnetic material may be a rare-earth magnet, which generally has strong attraction and repulsion forces and has high retentive capacity and resistance to demagnification. In certain embodiments, the rare-earth magnet is an alloy of neodymium, iron, and boron ("NdFeB"). NdFeB magnets may provide strong permanent magnetism, high retentive capacity, and resistance to demagnetization. In preferred embodiments, the magnetic material is a N52 NdFeB rare earth magnet.

In embodiments of the invention, the one or more magnets of the acetabular component are magnetized along the long axis of the magnet(s). For example, in embodiments in which the magnet(s) are generally cylindrical, the magnet(s) are magnetized along the axis that passes through the center of each circular end of the magnet. Alternatively, the one or more magnets of the acetabular component are magnetized in a radial orientation. In certain embodiments, magnet(s) at the central dome are magnetized axially along the length of the long axis of the magnet(s), and magnet(s) in an array surrounding the magnet(s) at central dome are magnetized axially along the length of the long axis of the magnet(s) or are magnetized in a radial orientation.

In some embodiments, each magnet may comprise the magnetic material and a casing that encloses the magnetic material. The casing will prevent the magnet from exposure to the environment. The magnetic material may be hermetically sealed within the casing. In some embodiments, the casing may comprise two or more components (e.g., an upper component and a lower component), in which the two or more components may be attached together (e.g., by laser-welding) in order to create a hermetically-sealed environment for the magnetic material.

The casing may be fabricated with a metal alloy known in the art for orthopaedic applications, for example, titanium, cobalt chromium, or stainless steel. In certain embodiments, the casing or plate may comprise a polymer, such as polyetheretherketone (PEEK) or polyurethane, or a combination thereof. In alternative embodiments, the casing or plate may comprise composites of polymers and fibers, such as carbon fiber-reinforced PEEK.

The shape of the casing may be primarily determined by the shape of the magnetic material within the casing. In some embodiments, the casing may comprise the same general shape as the magnetic material. For example, if the magnetic material is generally cylindrical, the casing may also be generally cylindrical; if the magnetic material is generally disc-shaped, the casing may also be generally disc-shaped. In some embodiments, the casing may be in the form of a screw, which may be compatible for use with magnetic material that is generally cylindrical, prism-shaped, conal, or pyramidal.

The casing may comprise an exterior surface that faces the outer environment, and an interior surface that faces the magnetic material. The exterior surface may be smooth or may comprise surface modifications that stabilize and/or prevent movement, such as rotation, of the magnet positioned in the acetabular component. In some embodiments, the surface modifications may adhere the magnet to the acetabular component or may generate friction between the magnet and the acetabular component. The surface modifications may comprise a roughened surface or a pattern of protrusions that are raised from the surface. The surface modifications may also comprise screw thread(s) or a grooved design, such as in embodiments in which the casing is in the form of a screw, or any other acceptable surgical configuration.

The magnet may comprise a size appropriate for use in the acetabular component of a THR prosthesis and for generating the desired magnetic force to prevent dislocation between the acetabular component and the femoral component. For example, in embodiments in which the magnets are generally cylindrical, the magnets may have a diameter of about 2 mm to about 20 mm, or about 3 mm to about 18 mm, or about 4 mm to about 15 mm, or any diameter therebetween; and a length of about 2 mm to about 15 mm, or about 3 mm to about 10 mm, or any length therebetween. In some embodiments, the size of the cylindrical magnet may depend on whether the magnet is positioned at the dome of the acetabular component or at the intermediate walls of the acetabular component. For example, cylindrical magnets positioned at the dome of the acetabular component may have a diameter of about 4 mm to about 18 mm, or about 6 mm to about 14 mm, or any diameter therebetween; and cylindrical magnets positioned at the intermediate walls of the acetabular component may have a diameter of about 3 mm to about 15 mm, or about 5 mm to about 10 mm, or any diameter therebetween.

In embodiments in which the central magnet **13'** is a larger magnet, i.e., about 15 mm to about 20 mm in diameter and/or about 10 mm to about 15 mm in length, the curvature of the outer surface **5** may be flattened at the central dome **10** to accommodate the larger central magnet **13'.** The flattened curvature may be accompanied by a reduced shell thickness **4** and/or, in embodiments in which the acetabular component **1** also comprises a liner **6,** a reduced liner thickness **8.**

In embodiments of the invention, and as demonstrated in Figures 2A and 2B, the femoral component **21** of the THR prosthesis may comprise a stem portion **22** that comprises a proximal end **23** and a distal end **24;** a neck portion **25** that comprises a tapered end **26** and a base end **27;** and a spherical head **28.** The neck portion **25** extends at an angle from the stem portion **22,** in which the neck portion **25** and the stem portion **22** are joined at the base end **27** of the neck portion **25** and the proximal end **23** of the stem portion **22.** The spherical head **28** may be affixed to the tapered end **26** of the neck portion **25,** and may comprise one or more magnets **34.**

The cross-sectional area of the neck portion **25** may decrease towards its tapered end **26,** such that cross-sectional area at the base end **27** is greater than the cross-sectional area at the tapered end **26.**

The spherical head **28** may comprise a tapered volume **31,** an outside surface **29,** and a thickness **30** between the outside surface **29** and the tapered volume **31.** The tapered volume **31** extends inward from the outside surface **29** of the spherical head **28** and is configured to receive all or part of the neck portion **25.** The tapered volume **31** is defined by an end surface **32** that is the innermost edge of the tapered volume **31,** and walls **33** that extend from the outside surface **29** to the end surface **32.** The cross-sectional area of the tapered volume **31** may decrease towards the end surface **32,** such that the cross-sectional area of the tapered volume **31** near the outside surface **29** is greater than the cross-sectional area of the tapered volume **31** at the end surface **32.**

The femoral component **21** may comprise materials known in the art for femoral components **21** of THR prostheses. For instance, the femoral component **21** may comprise a metal such as stainless steel, titanium, chromium, cobalt, or a combination thereof; a plastic such as polyethylene or cross-linked polyethylene; or a ceramic. Notably, the stem portion **22,** the neck portion **25,** and the spherical head **28** of the femoral component **21** may comprise different materials. For example, the stem portion **22** and the neck portion **25** may comprise a metal while the spherical head **28** may comprise a ceramic.

The one of more magnets **34** of the femoral component **21** may be positioned at the end surface **32** of the tapered volume **31,** at the walls **33** of the tapered volume **31,** embedded into the thickness **30** of the spherical head **28** adjacent to the end surface **32** of the tapered volume **31,** embedded into the thickness **30** of the spherical head **28** adjacent to the walls 33 of the tapered volume **31,** or a combination thereof. In certain embodiments, the one of more magnets **34** are positioned at the end surface **32** of the tapered volume **31.** In certain embodiments, the one of more magnets **34** are positioned adjacent to the end surface **32** of the tapered volume **31.** In certain embodiments, the one of more magnets **34** are positioned at both the end surface **32** and adjacent to the end surface **32** of the tapered volume **31.**

The number of magnets and their position in the femoral component may vary. For example, in some embodiments, a single magnet **34'** may be positioned at or adjacent to the center of the end surface **32** of the tapered volume **31** of the spherical head **28;** an example of such an arrangement is demonstrated in Figures 3A to 4B. In some embodiments, an array of magnets **34"** may be positioned at or adjacent to the end surface **32** of the tapered volume **31** of the spherical head **28,** in which each magnet in the array **34"** is positioned at an approximately equal distance from the center of the end surface **32** and/or is positioned at an approximately equal distance from each other. The array **34"** may comprise 2 to 16 magnets, or 4 to 12 magnets, or a number therebetween, such as 2 magnets, or 3 magnets, or 4 magnets, or 5 magnets, or 6 magnets, or 7 magnets, or 8 magnets, or 9 magnets, or 10 magnets, or 11 magnets, or 12 magnets, or 13 magnets, or 14 magnets, or 15 magnets, or 16 magnets.

In some embodiments, magnets may be positioned at the end surface **32** of the tapered volume **31** of the spherical head **28,** in which one magnet **34'** is at or adjacent to the center of the end surface **32** (i.e., a "central magnet") and an array of magnets **34"** surround the central magnet **34'** and are positioned at an approximately equal distance from the central magnet **34'** and/or is positioned at an approximately equal distance from each other; an example of such an arrangement is demonstrated in Figures 5A through 8B. In some embodiments, the magnets **34"** surrounding the central magnet **34'** are embedded in the thickness **30** of the spherical head **28.** In some embodiments, the array **34"** may comprise 2 to 16 magnets, or 4 to 12 magnets, or a number therebetween, such as 2 magnets, or 3 magnets, or 4 magnets, or 5 magnets, or 6 magnets, or 7 magnets, or 8 magnets, or 9 magnets, or 10 magnets, or 11 magnets, or 12 magnets, or 13 magnets, or 14 magnets, or 15 magnets, or 16 magnets.

In embodiments in which the femoral component **21** comprises a central magnet **34'** at the end surface **31,** the central magnet **34'** may be oriented such that the long axis of the magnet **34'** is perpendicular to the end surface **31.**

In embodiments in which the femoral component **21** comprises a central magnet **34'** at the end surface **31,** and the acetabular component **1** comprises a central magnet **13'** at the central dome **10,** the central magnet **34'** of the femoral component **21** may be oriented such that the long axis of the central magnet **34'** is parallel to the long axis of the central magnet **13'** of the acetabular component **1.**

In embodiments in which an array of magnets **34"** surround a central magnet **34',** the long axis of the surrounding magnets **34"** may be parallel with the long axis of the central magnet **34',** as demonstrated in Figures 5A and 5B. Alternatively, the long axis of the surrounding magnets **34"** may be angled to the long axis of the central magnet **34',** as demonstrated in Figures 6A and 8B. This angle may be about 10° to about 80°, or about 20° to about 70°, or about 30° to about 60°, or any angle therebetween, such as about 35°, or about 40°, or about 45°, or about 50°.

In some embodiments, the number of magnets **34** in the spherical head **28** may be the same as the number of magnets **13** in the acetabular component **1.** In some embodiments, the magnets **34** in the spherical head **28** are numbered and positioned such that, upon implantation into a subject in which the spherical head **28** is in articulation with the acetabular component **1,** when the leg is in a rest position, there is a corresponding magnet **13** in the acetabular component **1** for each magnet **34** in the spherical head **28.** In certain embodiments, the long axis of each magnet **34** in the spherical head **28** is aligned with the long axis of its corresponding magnet **13** in the acetabular component **1.** In certain embodiments in which the acetabular component **1** includes a magnet **13'** at the central dome **10** and the femoral component **21** includes a magnet **34'** at the end surface **32** of the tapered volume **31,** when the leg is in a rest position, the long axis of the magnet **13'** at the central dome **10** and the long axis of the magnet **34'** at the end surface **32** of the tapered volume **31** are aligned. As used herein, "rest position" or "resting position" means that the leg is not abducted or adducted, not flexed or hyperextended, and not rotated relative to the hip, i.e., a 0° hip position. In some embodiments, the "rest position" or "resting position" is consistent to when an individual is in a typical standing or laying position with the feet.

In some embodiments, the one or more magnets **34** in the femoral component **21** may be positioned such that the distance between the one or more magnets **34** and the outer surface **29** of the spherical head **28** may be no more than about 5 mm, or no more than about 4 mm, or no more than about 3 mm, or no more than about 1 mm.

In some embodiments, the one or more magnets **34** in the femoral component **21** may also be positioned such that the distance between the one or more magnets **34** in the spherical head **28** and the one or more magnets **13** in the acetabular component **1** may be no more than about 10 mm, or no more than about 9 mm, or no more than about 8 mm, or no more than about 7 mm, or no more than about 6 mm, or no more than about 5 mm, or no more than about 4 mm, or no more than about 3 mm, or no more than about 2 mm. In certain embodiments in which the acetabular component **1** includes a magnet **13'** at the central dome **10** and the femoral component **21** includes a magnet **34'** at the end surface **32** of the tapered volume **31,** the distance between the one or more magnets **13** of the acetabular component **1** and the one or more magnets **34** of the femoral component may be determined by the distance between the magnet **13'** at the central dome **10** of the acetabular component **1** and the magnet **34'** at the end surface **32** of the tapered volume **31** of the femoral component **21.** In certain embodiments, the distance may be determined when the long axis of the magnet **13'** at the central dome **10** of the acetabular component **1** is parallel to the long axis of the magnet **34'** at the end surface **32** of the tapered volume **31** of the femoral component **21.**

The one or more magnets **34** may be positioned in the spherical head **28** through means known in the art, including affixing by a press fit, screw designs (e.g., a screw with a magnet is screwed into the femoral component), taper lock, etc. In some embodiments, the one or more magnets **34** are positioned in the spherical head **28** via the outside surface **29** of the spherical head **28** (e.g., the magnet(s) are press-fitted, screwed into, locked in, etc., into the outside surface **29** of the spherical head **28**), via the end surface **32** of the tapered volume **31,** via the walls **33** of the tapered volume **31,** or a combination thereof. In some embodiments, the one or more magnets **34** may be embedded within the thickness **30** of the spherical head **28.**

The magnet(s) in the femoral component head may be generally any geometric shape, such as a cylinder, disc, prism (including rectangular prism, hexagonal prism, triangular prism, cube, etc.), cone, and pyramid. In certain embodiments, the one or more magnets are cylindrical. If more than one magnet is present, each magnet may comprise the same or a different geometric shape.

In embodiments in which the magnet is cylindrical or disc-shaped, the "long axis" refers to the line that is formed by the centers of the circular bases of the cylinder or disc. In embodiments in which the magnet is prism-shaped, the "long axis" refers to the line formed by the centers of bases in the direction of the longest dimension of the magnet. In embodiments in which the magnet is conal or pyramidal, the "long axis" refers to the line between the apex and the center of the base and perpendicular to the base.

In some embodiments, a surface of the magnet may comprise a geometric contour, such as a curvature, that is similar to the curvature of the spherical head.

The magnet(s) may comprise magnetic materials known in the art. For example, the magnetic materials may be iron-based, nickel-based, cobalt-based, or an alloy of rare-earth metals. In some embodiments, the magnetic material may be a rare-earth magnet, which generally has strong attraction and repulsion forces and has high retentive capacity and resistance to demagnification. In certain embodiments, the rare-earth magnet is NdFeB.

In embodiments of the invention, the one or more magnets of the femoral component are magnetized axially along the length of the long axis of the magnet(s). For example, in embodiments in which the magnet(s) are generally cylindrical, the magnet(s) are magnetized along the axis that passes through the center of each circular end of the magnet. Alternatively, the one or more magnets of the femoral component are magnetized in a radial orientation. In certain embodiments, magnet(s) at the center of the end surface **32** of the tapered volume **31** are magnetized axially along the length of the long axis of the magnet(s), and magnet(s) in an array surrounding the magnet(s) at center of the end surface **32** of the tapered volume **31** are magnetized axially along the length of the long axis of the magnet(s) or are magnetized in a radial orientation.

In some embodiments, each magnet may comprise the magnetic material and a casing that encloses the magnetic material. The casing will prevent the magnet from exposure to the environment. The magnetic material may be hermetically sealed within the casing. In some embodiments, the casing may comprise two or more components (e.g., an upper component and a lower component), in which the two or more components may be attached together (e.g., by laser-welding) in order to create a hermetically-sealed environment for the magnetic material.

The casing may be fabricated with a metal alloy known in the art for orthopaedic applications, for example, titanium, cobalt chromium, or stainless steel. In certain embodiments, the casing or plate may comprise a polymer, such as PEEK or polyurethane, or a combination thereof. In alternative embodiments, the casing or plate may comprise composites of polymers and fibers, such as carbon fiber-reinforced PEEK.

The shape of the casing may be primarily determined by the shape of the magnetic material within the casing. In some embodiments, the casing may comprise the same general shape as the magnetic material. For example, if the magnetic material is generally cylindrical, the casing may also be generally cylindrical; if the magnetic material is generally disc-shaped, the casing may also be generally disc-shaped. In some embodiments, the casing may be in the form of a screw, which may be compatible for use with magnetic material that is generally cylindrical, prism-shaped, conal, or pyramidal.

The casing may comprise an exterior surface that faces the outer environment, and an interior surface that faces the magnetic material. The exterior surface may be smooth or may comprise surface modifications that stabilize and/or prevent movement, such as rotation, of the magnet positioned in the spherical head. In some embodiments, the surface modifications may adhere the magnet to the spherical head or may generate friction between the magnet and the spherical head. The surface modifications may comprise a roughened surface or a pattern of protrusions that are raised from the surface. The surface modifications may also comprise screw thread(s) or a grooved design, such as in embodiments in which the casing is in the form of a screw, or any other acceptable surgical configuration.

The magnet may comprise a size appropriate for use in the spherical head of a femoral component of a THR prosthesis and for generating the desired magnetic force to prevent dislocation between the acetabular component and the femoral component. For example, in embodiments in which the magnets are generally cylindrical, the magnets may have a diameter of about 1 mm to about 30 mm, or about 3 mm to about 25 mm, or about 5 mm to about 20 mm, or any diameter therebetween; and a length of about 2 mm to about 30 mm, or about 3 mm to about 25 mm, or about 5 mm to about 20 mm, or any length therebetween.

In some embodiments, the size of the cylindrical magnet may depend on whether the magnet is positioned at the end surface **32** of the tapered volume **31** or in the thickness **30** of the spherical head **28.** For example, cylindrical magnets positioned at the end surface **32** of the tapered volume **31** may have a diameter of about 5 mm to about 30 mm, or about 10 mm to about 20 mm, or any diameter therebetween; while cylindrical magnets positioned in the thickness **30** of the spherical head **28** may have a diameter of about 1 mm to about 20 mm, or about 3 mm to about 10 mm, or any diameter therebetween.

### Use of the THR Prosthesis

The invention may be used in (i) a method of treating a subject in need of THR; (ii) a method of stabilizing a THR prosthesis in a subject; (iii) a method of reducing incidence of THR dislocation in a subject; (iv) a method of reducing risk of THR dislocation in a subject; (v) a method of reducing risk of THR dislocation due to impingement in a subject; (vi) a method of reducing risk THR subluxation in a subject; and (vii) a method of reducing osteolysis associated with THR in a subject. The invention may be used to (i) treat a subject in need of THR; (ii) stabilize a THR prosthesis in a subject undergoing THR; (iii) reduce incidence of THR dislocation in a subject; (iv) reduce risk of THR dislocation in a subject; (iv) reduce risk of THR dislocation due to impingement in a subject; (v) reduce risk of THR subluxation in a subject; and (vi) reduce osteolysis associated with THR in a subject. The invention may be suitable for use in (i) treating a subject in need of THR; (ii) stabilizing a THR prosthesis in a subject undergoing THR; (iii) reducing incidence of THR dislocation in a subject; (iv) reducing risk of THR dislocation in a subject; (v) reducing risk of THR dislocation due to impingement in a subject; (vi) reducing risk of THR subluxation in a subject; and (vii) reducing osteolysis associated with THR in a subject.

These methods or uses of the present invention may comprise implanting the THR prosthesis in the subject. Implantation of the THR prosthesis may comprise (a) removing all or a portion of the cartilage of the acetabulum and implanting the acetabular component; and (b) cutting the proximal femoral neck and implanting the femoral component. In some examples, implantation of the femoral component may comprise affixing all or part of the stem portion of the femoral component into the marrow cavity by, for example, press fit or using bone cement. The spherical head of the femoral component is fit into the inner surface of the shell or, if a liner is present, the inner surface of the liner.

### Methods of Manufacturing the THR Prosthesis

An aspect of the present invention relates to methods of manufacturing the THR prosthesis of the present invention. The method comprises preparing one or more bores in the acetabular component and one or more bores in the femoral component of a THR prosthesis, and inserting a magnet into each of the bores of the acetabular component and of the femoral component.

The THR prosthesis used in the method of manufacturing is a THR prosthesis as known in the art.

The one or more bores may be prepared by methods known in the art, such as using a drill or other known devices. Bores may be in a shape that accommodates the shape of the magnets that will be inserted therein; for example, a bore may be cylindrical to accommodate a cylindrical magnet.

The placement of the bores may be based on the position and orientation that is intended for the magnets that will subsequently be inserted into the bores in order to form the THR prosthesis of the present invention. For example, in examples in which the acetabular component comprises a central magnet at the central dome, a bore may be prepared at the location of the central dome and in a direction perpendicular to the tangent of the curvature at the central dome, e.g., a direction in which the long axis of the bore is perpendicular to the tangent of the curvature at the central dome. In embodiments in which the acetabular component additionally comprises an array of magnets surrounding the central magnet, bores for each of the magnets in the array may be prepared surrounding the bore prepared for the central magnet. These bores may be prepared through the outer surface of the shell, through the inner surface of the shell, through the outer surface of the liner, or a combination thereof. In certain embodiments, bores may be prepared in both the inner surface of the shell and the outer surface of the liner, in which the magnets after insertion will extend between the shell and the liner; such bores may be cylindrical or, alternatively, may be in the shape of a groove that accommodates a portion of a magnet.

In embodiments in which the femoral component comprises a central magnet at the end surface of the tapered volume, a bore may be prepared at the location of the end surface in a direction perpendicular to the end surface, e.g., a direction in which the long axis of the bore is perpendicular to the end surface. In embodiments in which the femoral component additionally comprises an array of magnets surrounding the central magnet, bores for each of the magnets in the array may be prepared surrounding the bore prepared for the central magnet. These bores may be prepared through the tapered volume, such as through the end surface, through the walls, or a combination thereof. Alternatively, or in addition, bores may be prepared through the outside surface of the spherical head.

In embodiments in which the magnet is cylindrical or disc-shaped, the "long axis" of the bore refers to the line that is formed by the centers of the circular bases of the cylinder or disc. In embodiments in which the bore is another shape such as a prism-shaped, the "long axis" refers to the line formed by the centers of bases in the direction of the longest dimension of the bore. In embodiments in which the bore is conal or pyramidal, the "long axis" refers to the line between the apex and the center of the base and perpendicular to the base.

A method of manufacturing the THR prosthesis of the present invention may not include preparing the bores; rather the method comprises inserting magnets into bores within the acetabular component and the femoral component of the prosthesis, in which the bores are positioned and oriented as described herein for the magnets in the THR prosthesis that comprises magnets according to embodiments of the present invention.

With this in mind, an example relates to a THR prosthesis that can accommodate magnets. The THR prosthesis may comprise an acetabular component with one or more bores, and a femoral component with one or more bores. These bores may be positioned and oriented as described herein for the magnets in the THR prosthesis that comprises magnets according to embodiments of the present invention (e.g., bores positioned at the central dome of the acetabular component, surrounding the central dome of the acetabular component, at end the surface in the femoral component, surrounding the end surface in the femoral component, etc.).

The magnets may be inserted into the bores by press fit. In some examples, a medical adhesive may be used to affix the magnets within the bores. In addition, an epoxy or similar medical adhesive may be used to fill the rest of the bore space once the magnet has been inserted. Such medical adhesives are known in the art.

### Kits

An aspect of the present disclosure relates to a kit that can be used to prepare the THR prosthesis of the present invention.

In some examples, the kit may comprise a THR prosthesis and two or more magnets. The THR prosthesis may be a THR prosthesis as is known in the art, and the two or more magnets may be in accordance with the magnets described herein.

In certain examples, the kit may further comprise a package insert. As used herein, "package insert" means a document that provides information on how to prepare the THR prosthesis of the present invention, for example, information based on the methods of manufacturing the THR prosthesis described herein. The package insert may further comprise safety information and other information required by a regulatory agency. A package insert can be a physical printed document in some embodiments.

In certain examples, the kit may further comprise one or more devices for preparing bores. Examples of such devices include, but are not limited to, a drill, a drill bit, and a combination thereof.

In certain examples, the kit may further comprise an adhesive for adhering the magnets after the magnets are inserted into the bores.

Alternatively, the kit may comprise a THR prosthesis that comprises bores in accordance with embodiments of the present invention, and two or more magnets. The bores may be positioned and oriented such that insertion of the magnets into the bores can result in the THR prosthesis that comprise magnets in accordance with embodiments of the present invention (e.g., bores positioned at the central dome of the acetabular component, surrounding the central dome of the acetabular component, at end the surface in the femoral component, surrounding the end surface in the femoral component, etc.).

In other examples, the kit may comprise a THR prosthesis that comprises magnets in accordance with embodiments of the present invention, and a package insert as described herein.

### EXAMPLES

### Example 1

Modeling was used to simulate forces in a THR prosthesis comprising an acetabular component with a single cylindrical magnet at the central dome and a femoral component with a single cylindrical magnet at the end surface of the tapered volume in the spherical head, as shown in Figures 3A and 3B. The cylindrical magnet in the acetabular component was 10 mm in diameter and 5 mm in length, and the cylindrical magnet in the spherical head was 12 mm in diameter and 12 mm in length. Each magnet was magnetized along its long axis and were oriented such that, when the acetabular component and the femoral component were in a 0° hip position, the long axis of the magnet in the femoral component was parallel with the long axis of the magnet in the acetabular component (see Figure 3A).

The forces were determined at two hip positions and two separation distances between the magnet in the acetabular component and the magnet in the femoral component. The two hip positions were the 0° hip position (see Figure 3A) and a 35° hip position, in which the long axis of the magnet in the femoral component is at a 35° angle relative to the long axis of the magnet in the acetabular component (such an angle can occur when the leg flexes and extends in a typical gait) (see Figure 3B). The two separation distances were 5 mm and 7 mm, which refers to the distance between the magnet in the acetabular component and the magnet in the femoral component at the 0° hip position.

The magnetic forces were modeled using JMAG^{®}, a simulation technology that utilizes finite element analysis to calculate the magnetic forces and fields.

Table 1 below provides the total force applied on the spherical head of the femoral component for each hip position and separation distance resulting from the attraction between the magnet of the acetabular component and the magnet of the femoral component. These results show that at the 0° hip position the total force on the spherical head was greater when the separation distance was smaller, but at the 35° hip position the total force on the spherical head was less when the separation distance was smaller.

**Table 1. Total force on the spherical head of the femoral component for each hip position and separation distance as a result of the attraction between the magnet of the acetabular component and the magnet of the femoral component (note: the angle and the arrow for the force at the 35° hip position represents the direction of the force).**

| **Hip Position** | **Separation Distance** | **Total Force** |
|---|---|---|
| 0° | 5 mm | 10.1 N |
| | 7 mm | 6.2 N |
| 35° | 5 mm | 0.96 N @ 51.7° ↘ |
| | 7 mm | 2.06 N @ 73.3° ↘ |

### Example 2

Modeling was used to simulate forces in a THR prosthesis comprising an acetabular component and a femoral component, in accordance with embodiments of the invention and as shown in Figures 4A and 4B. The acetabular component comprised a single cylindrical magnet at the central dome (i.e., a central magnet) and an array of cylindrical magnets surrounding the central magnet. Two different magnet arrays were studied: (i) a first array comprising four magnets equidistant from the central magnet and equidistant from each other (Figure 4A); and (ii) a second array comprising six magnets equidistant from the central magnet and equidistant from each other (Figure 4B). The central magnet was 10 mm in diameter and 5 mm in length, and the surrounding magnets were 6 mm in diameter and 5 mm in length. The magnets in the array were positioned such that the long axis of each of these magnets are at a 35° angle to the long axis of the central magnet.

The femoral component contained a single cylindrical magnet at the end surface of the tapered volume in the spherical head. The single magnet was 12 mm in diameter and 12 mm in length, and was oriented such that its long axis was parallel with the long axis of the central magnet in the acetabular component when the acetabular component and the femoral component were in a 0° hip position.

Each magnet in the acetabular and femoral component was magnetized along its long axis.

The forces were determined at the 0° hip position and at two separation distances-5 mm and 7 mm-between the central magnet in the acetabular component and the single magnet in the femoral component. The magnetic forces were modeled using JMAG^{®}.

Table 2 below provides the total force applied on the spherical head of the femoral component for each array of cylindrical magnets surrounding the single magnet in the acetabular component and for each separation distance resulting from the attraction between the magnets of the acetabular component and the magnet of the femoral component. These results show that, at a separation distance of 5 mm, the total force on the spherical head was greater when the acetabular component had an array of four magnets surrounding the central magnet as compared to when the acetabular component had an array of six magnets surrounding the central magnet. On the other hand, at a separation distance of 7 mm, the total force on the spherical head was the same when the acetabular component had an array of four magnets surrounding the central magnet as compared to when the acetabular component had an array of six magnets surrounding the central magnet. Further, for each array of the acetabular component, the total force on the spherical head was greater when the separation distance was less.

**Table 2. Total force applied on the spherical head of the femoral component for each array of cylindrical magnets surrounding the single magnet in the acetabular component and for each separation distance resulting from the attraction between the magnets of the acetabular component and the magnet of the femoral component.**

| **Number of Array Magnets** | **Separation Distance** | **Total Force** |
|---|---|---|
| 4 | 5 mm | 9.1 N |
| | 7 mm | 5.4 N |
| 6 | 5 mm | 8.2 N |
| | 7 mm | 5.4 N |

### Example 3

Modeling was used to simulate forces in a THR prosthesis comprising an acetabular component and a femoral component, in accordance with embodiments of the invention and as shown in Figures 5A and 5B. The acetabular component contained a single cylindrical magnet at the central dome (i.e., a central magnet) surrounded by an array of four cylindrical magnets equidistant from the central magnet and equidistant from each other. The central magnet was 10 mm in diameter and 5 mm in length, and the magnets in the array were 6 mm in diameter and 5 mm in length. The magnets in the array were positioned such that the long axis of these magnets are at a 35° angle to the long axis of the central magnet. Each magnet was magnetized along its long axis.

The femoral component comprised a single cylindrical magnet at the center of the edge surface (i.e., a central magnet) surrounded by an array of four cylindrical magnets equidistant from the central magnet and from each other. The central magnet was 12 mm in diameter and 12 mm in length, and the surrounding magnets were 6 mm in diameter and 14 mm in length. The central magnet was oriented such that its long axis was parallel with the long axis of the central magnet in the acetabular component when the acetabular component and the femoral component were in a 0° hip position. The long axis of the magnets in the array were parallel to the long axis of the central magnet. Each magnet was magnetized along its long axis.

The forces were determined at two hip positions and two separation distances between the central magnet in the acetabular component and the central magnet in the femoral component. The two hip positions were the 0° hip position (see Figure 5A) and a 35° hip position, in which the long axis of the magnet in the femoral component is at a 35° angle relative to the long axis of the magnet in the acetabular component (see Figure 3B). The two separation distances were 5 mm and 7 mm, which refers to the distance between the central magnet in the acetabular component and the central magnet in the femoral component at the 0° hip position. The magnetic forces were modeled using JMAG^{®}.

Table 3 below provides the total force applied on the spherical head of the femoral component for each hip position and separation distance resulting from the attraction between the magnets of the acetabular component and the magnets of the femoral component. These results show that, at both hip positions, the total force on the spherical head at the separation distance of 5 mm was greater than the total force on the spherical head at the separation distance of 7 mm. At both separation distances, the total force on the spherical head at the 0° hip position was greater than at the 35° hip position. In addition, comparing these total forces on the spherical head with those described in Example 1 and shown in Table 1, the presence of the magnet array increased the total force on the spherical head for each separation distance and each hip position.

**Table 3. Total force applied on the spherical head of the femoral component for each hip position and separation distance resulting from the attraction between the magnets of the acetabular component and the magnets of the femoral component (note: the angle and the arrow for the attraction force at the 35° hip position represents the direction of the force).**

| **Hip Position** | **Separation Distance** | **Total Force** |
|---|---|---|
| 0° | 5 mm | 12.5 N |
| | 7 mm | 8.0 N |
| 35° | 5 mm | 5.49 N @ 12.8° ↘ |
| | 7 mm | 4.38 N @ 22.1° ↘ |

### Example 4

Modeling was used to simulate forces in a THR prosthesis comprising an acetabular component and a femoral component, in accordance with embodiments of the invention and as shown in Figures 6A-7B.

Two different THR prostheses were studied. In the first THR prosthesis, the acetabular component contained a single cylindrical magnet at the central dome (i.e., a central magnet) and an array of four cylindrical magnets surrounding and equal distant from the central magnet and equidistant from each other, in which the magnets in the array were positioned such that the long axis of these magnets are at a 35° angle to the long axis of the central magnet (Figures 6A and 6B). The femoral component contained a single cylindrical magnet at the end surface of the tapered volume (i.e., a central magnet) and an array of four cylindrical magnets surrounding and equal distant from the central magnet and equidistant from each other, in which the central magnet was oriented such that its long axis was parallel with the long axis of the central magnet in the acetabular component when the acetabular component and the femoral component were in a 0° hip position, and the magnets in the array were positioned such that the long axis of these magnets were at a 35° angle to the long axis of the central magnet (Figures 6A and 6B).

In the second THR prosthesis, the acetabular component contained a single cylindrical magnet at the central dome (i.e., a central magnet) and an array of four cylindrical magnets surrounding and equal distant from the central magnet and equidistant from each other, in which the magnets in the array were positioned such that the long axis of these magnets are at a 50° angle to the long axis of the central magnet (Figures 7A and 7B). The femoral component contained a single cylindrical magnet at the end surface of the tapered volume (i.e., a central magnet) and an array of four cylindrical magnets surrounding and equal distant from the central magnet and equidistant from each other, in which the central magnet was oriented such that its long axis was parallel with the long axis of the central magnet in the acetabular component when the acetabular component and the femoral component were in a 0° hip position, and the magnets in the array were positioned such that the long axis of these magnets were at a 50° angle to the long axis of the central magnet (Figures 7A and 7B).

For both THR prostheses, in the acetabular component, the central magnet was 10 mm in diameter and 5 mm in length, and the magnets in the array were 6 mm in diameter and 5 mm in length; and in the femoral component, the central magnet was 12 mm in diameter and 12 mm in length, and the magnets in the array were 6 mm in diameter and 14 mm in length. Each magnet of both THR prosthesis was magnetized along its long axis.

For the first THR prosthesis, the forces were determined at two hip positions and two separation distances between the central magnet in the acetabular component and the central magnet in the femoral component. The two hip positions were the 0° hip position (see Figure 6A) and a 35° hip position, in which the long axis of the magnet in the femoral component is at a 35° angle relative to the long axis of the magnet in the acetabular component (see Figure 6B). The two separation distances were 5 mm and 7 mm, which refers to the distance between the central magnet in the acetabular component and the central magnet in the femoral component at the 0° hip position.

For the second THR prosthesis, the forces were also determined at two hip positions and two separation distances between the central magnet in the acetabular component and the central magnet in the femoral component. The two hip positions were the 0° hip position (see Figure 7A) and a 50° hip position, in which the long axis of the magnet in the femoral component is at a 50° angle relative to the long axis of the magnet in the acetabular component (see Figure 7B). The two separation distances were 5 mm and 7 mm, which refers to the distance between the central magnet in the acetabular component and the central magnet in the femoral component at the 0° hip position.

The magnetic forces were modeled using JMAG^{®}.

Table 4 below provides for each THR prosthesis the total force applied on the spherical head of the femoral component for each hip position and separation distance resulting from the attraction between the magnets of the acetabular component and the magnets of the femoral component. These results show that, for both THR prostheses, the total force on the spherical head was greater at the separation distance of 5 mm as compared to 7 mm, and was greater at the 0° hip position as compared to the angled hip position. In addition, at each separation distance and hip position, the total force on the spherical head was greater for the second THR prosthesis, which contained a 50° angle between the long axes of the magnets in the array and the long axis of the central magnet in both the acetabular and femoral components, as compared to the first THR prosthesis, which contained a 35° angle between the long axes of the magnets in the array and the long axis of the central magnet in both the acetabular and femoral components.

**Table 4. Total force applied on the spherical head of the femoral component for each hip position and separation distance resulting from the attraction between the magnets of the acetabular component and the magnets of the femoral component, for both the first and second THR prostheses (note: the angle and the arrow for the attraction force at the 35° hip position and 50° hip position represents the direction of the force).**

| **THR Prosthesis** | **Hip Position** | **Separation Distance** | **Total Force** |
|---|---|---|---|
| First THR Prosthesis (35° array angle) | 0° | 5 mm | 11.69 N |
| First THR Prosthesis (35° array angle) | 0° | 7 mm | 7.21 N |
| First THR Prosthesis (35° array angle) | 35° | 5 mm | 4.39 N @ 9.4° ↘ |
| First THR Prosthesis (35° array angle) | 35° | 7 mm | 3.28 N @ 17.0° ↘ |
| Second THR Prosthesis (50° array angle) | 0° | 5 mm | 15.63 N |
| Second THR Prosthesis (50° array angle) | 0° | 7 mm | 9.09 N |
| Second THR Prosthesis (50° array angle) | 50° | 5 mm | 6.68N@27.1° ↘ |
| Second THR Prosthesis (50° array angle) | 50° | 7 mm | 3.70 N @ 26.3° ↘ |

### Example 5

Modeling was used to simulate forces in a THR prosthesis comprising an acetabular component and a femoral component, in accordance with embodiments of the invention and as shown in Figures 8A and 8B. The acetabular component contained a single cylindrical magnet at the central dome (i.e., a central magnet) surrounded by an array of four cylindrical magnets equidistant from the central magnet and equidistant from each other. The central magnet was 15 mm in diameter and 5 mm in length, and the surrounding magnets were 10 mm in diameter and 5 mm in length. The magnets in the array were positioned such that the long axis of these magnets are at a 50° angle to the long axis of the central magnet. Each magnet was magnetized along its long axis.

The femoral component contained a single cylindrical magnet at the center of the end surface (i.e., a central magnet) surrounded by an array of four cylindrical magnets equidistant from the central magnet and equidistant from each other. The central magnet was 12 mm in diameter and 12 mm in length, and the surrounding magnets were 6 mm in diameter and 14 mm in length. The central magnet was oriented such that its long axis was parallel with the long axis of the central magnet in the acetabular component when the acetabular component and the femoral component were in a 0° hip position. The magnets in the array were positioned such that the long axis of these magnets are at a 50° angle to the long axis of the central magnet. Each magnet was magnetized along its long axis.

The forces were determined at two hip positions and two separation distances between the central magnet in the acetabular component and the central magnet in the femoral component. The two hip positions were the 0° hip position (see Figure 8A) and a 50° hip position, in which the long axis of the magnet in the femoral component is at a 35° angle relative to the long axis of the magnet in the acetabular component (see Figure 8B). The two separation distances were 5 mm and 7 mm, which refers to the distance between the central magnet in the acetabular component and the central magnet in the femoral component at the 0° hip position. The magnetic forces were modeled using JMAG^{®}.

Table 5 below provides the total force applied on the spherical head of the femoral component for each hip position and separation distance resulting from the attraction between the magnets of the acetabular component and the magnets of the femoral component. These results show that, at both hip positions, the total force on the spherical head at the separation distance of 5 mm was greater than the total force on the spherical head at the separation distance of 7 mm. At both separation distances, the total force on the spherical head at the 0° hip position was greater than at the 50° hip position.

In addition, comparing these forces with those described in Example 4 and shown in Table 4, the use of larger magnets in the acetabular component resulted in a greater total force on the spherical head for each separation distance and each hip position.

**Table 5. Total force applied on the spherical head of the femoral component for each hip position and separation distance resulting from the attraction between the magnets of the acetabular component and the magnets of the femoral component (note: the angle and the arrow for the attraction force at the 50° hip position represents the direction of the force).**

| **Hip Position** | **Separation Distance** | **Total Force** |
|---|---|---|
| 0° | 5 mm | 25.20 N |
| | 7 mm | 17.37 N |
| 50° | 5 mm | 11.52 N @ 27.0° ↘ |
| | 7 mm | 7.01 N @ 21.0° ↘ |

The foregoing description is given for clearness of understanding only, and no unnecessary limitations should be understood therefrom, as modifications within the scope of the invention may be apparent to those having ordinary skill in the art.

Detailed examples of the present methods and magnetic devices are disclosed herein; however, it is to be understood that the disclosed embodiments are merely illustrative and that the methods and magnetic devices may be embodied in various forms. In addition, each of the examples given in connection with the various examples of the systems and methods are intended to be illustrative, and not restrictive.

Throughout this specification and the claims which follow, unless the context requires otherwise, the word "comprise" and variations such as "comprises" and "comprising" will be understood to imply the inclusion of a stated integer or step or group of integers or steps but not the exclusion of any other integer or step or group of integers or steps.

Throughout the specification, where compositions are described as including components or materials, it is contemplated that the compositions can also consist essentially of, or consist of, any combination of the recited components or materials, unless described otherwise. Likewise, where methods are described as including particular steps, it is contemplated that the methods can also consist essentially of, or consist of, any combination of the recited steps, unless described otherwise. The invention illustratively disclosed herein suitably may be practiced in the absence of any element or step which is not specifically disclosed herein.

The practice of a method disclosed herein, and individual steps thereof, can be performed manually and/or with the aid of or automation provided by electronic equipment. Although processes have been described with reference to particular embodiments, a person of ordinary skill in the art will readily appreciate that other ways of performing the acts associated with the methods may be used. For example, the order of various steps may be changed, unless described otherwise. In addition, some of the individual steps can be combined, omitted, or further subdivided into additional steps.

## Claims

1. A total hip replacement (THR) prosthesis, comprising
(a) an acetabular component (1) having a full or partial hemispherical shape that comprises a central dome (10), a periphery (12), and an intermediate wall (110) therebetween, wherein the acetabular component (1) comprises a shell (2) and one or more magnets (13); and wherein the shell (2) comprises a concave inner surface (3), a convex outer surface (5), and a thickness (4) therebetween; and
(b) a femoral component (21) comprising
(i) a stem portion (22) comprising a proximal end (23) and a distal end (24),
(ii) a neck portion (25) comprising a tapered end (26) and a base end (27), wherein the base end (27) of the neck portion (25) is joined to the proximal end (23) of the stem portion (22), and the neck portion (25) extends at an angle from the stem portion (22), and
(iii) a spherical head (28) that is configured to be affixed to the tapered end (26) of the neck portion (25), wherein the spherical head (28) comprises one or more magnets (34);
wherein the spherical head (28) comprises a tapered volume (31), an outside surface (29), and a thickness (30) between the tapered volume (31) and the outside surface (29), and wherein the tapered volume (31) is configured to receive the tapered end (26) of the neck portion (25);
wherein the acetabular component (1) is configured to receive all or a portion of the spherical head (28) of the femoral component (21); and
wherein the one or more magnets (13) of the acetabular component (1) and the one or more magnets (34) of the spherical head 28 of the femoral component 21 are oriented to generate an attractive force therebetween, **characterised in that** the one or more magnets (13) comprise a single magnet (13') at the central dome (10) and an array of magnets (13") that surround the single magnet (13') at the central dome (10), wherein the magnets (13") in the array are equidistant to the single magnet (13') at the central dome (10).

2. The THR prosthesis of claim 1, wherein, in the acetabular component (1), the long axis of the single magnet (13') at the central dome (10) is perpendicular to the tangent line of the curvature at the central dome (10).

3. The THR prosthesis of claim 1 or 2, wherein, in the acetabular component (1), the array of magnets (13") surrounding the single magnet (13') comprise 2 to 16 magnets.

4. The THR prosthesis of claim 1, wherein, in the acetabular component (1), the magnets in the array (13") surrounding the single magnet (13') at the central dome (10) are equidistant from each other.

5. The THR prosthesis of claim 3 or 4, wherein, in the acetabular component (1), the magnets in the array (13") surrounding the single magnet (13') are oriented such that the long axes of the magnets in the array (13") are parallel to the long axis of the single magnet (13').

6. The THR prosthesis of claim 3 or 4, wherein, in the acetabular component (1), the magnets in the array (13") surrounding the single magnet (13') at the central dome (10) are oriented such that the long axes of the magnets in the array (13") are angled to the long axis of the single magnet (13').

7. The THR prosthesis of any one of claims 1-6, wherein, in the femoral component (21), the tapered volume (31) extends inward from the outside surface (29) of the spherical head (28),
wherein the tapered volume (31) is defined by an end surface (32) that is the innermost surface of the tapered volume (31), and walls (33) that extend from the outside surface (29) to the end surface (32), and
wherein the cross-sectional area of the tapered volume (31) decreases towards the end surface (32) from the outside surface (29) of the spherical head (28).

8. The THR prosthesis of any one of claim 7, wherein, in the femoral component (21), the one or more magnets (34) are at the end surface (32) of the tapered volume (31), at the walls (33) of the tapered volume (31), embedded into the thickness (30) of the spherical head (28) adjacent to the end surface (32) of the tapered volume (31), embedded into the thickness (30) of the spherical head (28) adjacent to the walls (33) of the tapered volume (31), or a combination thereof.

9. The THR prosthesis of claim 7 or 8, wherein, in the femoral component (21), the one or more magnets (34) comprise a single magnet (34') at the end surface (32), an array of magnets (34") at the end surface (32), or both a single magnet (34') at the end surface (32) and an array of magnets (34") surrounding the single magnet (34').

10. The THR prosthesis of any one of claims 7-9, wherein, in the femoral component (21), the single magnet (34') at the end surface (32) is oriented such that the long axis of the single magnet (34') is perpendicular to the end surface (32), or is oriented such that the long axis of the single magnet (34') in the femoral component (21) is parallel to the single magnet (13') in the acetabular component (1) when the total hip replacement prosthesis is in a 0° hip position.

11. The THR prosthesis of any one of claims 7-10, wherein, in the femoral component (21), the one or more magnets (34) comprise a single magnet (34') at the end surface (32) and an array of magnets (34") surrounding the single magnet (34'), and the magnets in the array (34") surrounding the single magnet (34') are equidistant from the single magnet (34'), are equidistant from each other, or a combination thereof.

12. The THR prosthesis of claim 11, wherein, in the femoral component (21), the magnets in the array (34") surrounding the single magnet (34') at the end surface (32) are oriented such that the long axes of the magnets in the array (34") are parallel to the long axis of the single magnet (34').

13. The THR prosthesis of claim 11, wherein, in the femoral component (21), the magnets in the array (34") surrounding the single magnet (34') at the end surface (32) are oriented such that the long axes of the magnets in the array (34") are angled to the long axis of the single magnet (34').

14. The THR prosthesis of any one of claims 1-13 for use in treating a subject in need of a THR.

15. A total hip replacement (THR) prosthesis, comprising
(a) an acetabular component (1) having a full or partial hemispherical shape that comprises a central dome (10), a periphery (12), and an intermediate wall (11) therebetween, wherein the acetabular component (1) comprises a shell (2) and one or more bores; wherein the one or more bores comprise a single bore at the central dome (10) and an array of bores that surround the single bore at the central dome (10), wherein the bores in the array are equidistant to the single bore at the central dome (10); and wherein the shell (2) comprises a concave inner surface (3), a convex outer surface (5), and a thickness (4) therebetween; and
(b) a femoral component (21) comprising
(i) a stem portion (22) comprising a proximal end (23) and a distal end (24),
(ii) a neck portion (25) comprising a tapered end (26) and a base end (27), wherein the base end (27) of the neck portion (25) is joined to the proximal end (23) of the stem portion (22), and the neck portion (25) extends at an angle from the stem portion (22), and
(iii) a spherical head (28) that is configured to be affixed to the tapered end (26) of the neck portion (25), wherein the spherical head (28) comprises one or more bores;
wherein the spherical head (28) comprises a tapered volume (31), an outside surface (29), and a thickness (30) between the tapered volume (31) and the outside surface (29), and wherein the tapered volume (31) is configured to receive the tapered end (26) of the neck portion (25);
wherein the acetabular component (1) is configured to receive all or a portion of the spherical head (28) of the femoral component (21); and
wherein each of the one or more bores in the shell of the acetabular component and each of the one or more bores n the spherical head are configured to receive a magnet.

## Patentansprüche

1. Totalhüftersatz (Total Hip Replacement (THR))-Prothese, umfassend
(a) eine Hüftgelenkpfannenkomponente (1), die eine vollständige oder teilweise halbkugelförmige Form aufweist, die eine zentrale Wölbung (10), einen Umfang (12) und eine Zwischenwand (110) dazwischen umfasst, wobei die Hüftgelenkpfannenkomponente (1) eine Schale (2) und einen oder mehrere Magnete (13) umfasst; und wobei die Schale (2) eine konkave Innenfläche (3), eine konvexe Außenfläche (5) und eine Dicke (4) dazwischen umfasst; und
(b) eine Oberschenkelkomponente (21), umfassend
(i) einen Schaftabschnitt (22), der ein proximales Ende (23) und ein distales Ende (24) umfasst,
(ii) einen Halsabschnitt (25), der ein verjüngtes Ende (26) und ein Basisende (27) umfasst, wobei das Basisende (27) des Halsabschnitts (25) mit dem proximalen Ende (23) des Schaftabschnitts (22) verbunden ist und sich der Halsabschnitt (25) in einem Winkel von dem Schaftabschnitt (22) erstreckt, und
(iii) einen kugelförmigen Kopf (28), der dazu konfiguriert ist, an dem verjüngten Ende (26) des Halsabschnitts (25) befestigt zu werden, wobei der kugelförmige Kopf (28) einen oder mehrere Magnete (34) umfasst;
wobei der kugelförmige Kopf (28) ein verjüngtes Volumen (31), eine Außenfläche (29) und eine Dicke (30) zwischen dem verjüngten Volumen (31) und der Außenfläche (29) umfasst und wobei das verjüngte Volumen (31) dazu konfiguriert ist, das verjüngte Ende (26) des Halsabschnitts (25) aufzunehmen;
wobei die Hüftgelenkpfannenkomponente (1) dazu konfiguriert ist, den gesamten kugelförmigen Kopf (28) der Oberschenkelkomponente (21) oder einen Teil davon aufzunehmen; und
wobei der eine oder die mehreren Magnete (13) der Hüftgelenkpfannenkomponente (1) und der eine oder die mehreren Magnete (34) des kugelförmigen Kopfs (28) der Oberschenkelkomponente (21) dazu ausgerichtet sind, eine Anziehungskraft dazwischen zu erzeugen, **dadurch gekennzeichnet, dass** der eine oder die mehreren Magnete (13) Folgendes umfassen:
einen einzelnen Magneten (13') an der zentralen Wölbung (10) und eine Anordnung von Magneten (13"), die den einzelnen Magneten (13') an der zentralen Wölbung (10) umgeben, wobei die Magnete (13") in der Anordnung den gleichen Abstand zu dem einzelnen Magneten (13') an der zentralen Wölbung (10) aufweisen.

2. THR-Prothese nach Anspruch 1, wobei in der Hüftgelenkpfannenkomponente (1) die Längsachse des einzelnen Magneten (13') an der zentralen Wölbung (10) senkrecht zu der Tangentenlinie der Krümmung an der zentralen Wölbung (10) ist.

3. THR-Prothese nach Anspruch 1 oder 2, wobei in der Hüftgelenkpfannenkomponente (1) die Anordnung von Magneten (13"), die den einzelnen Magneten (13') umgeben, 2 bis 16 Magnete umfasst.

4. THR-Prothese nach Anspruch 1, wobei in der Hüftgelenkpfannenkomponente (1) die Magnete in der Anordnung (13"), die den einzelnen Magneten (13') an der zentralen Wölbung (10) umgeben, den gleichen Abstand zueinander aufweisen.

5. THR-Prothese nach Anspruch 3 oder 4, wobei in der Hüftgelenkpfannenkomponente (1) die Magnete in der Anordnung (13"), die den einzelnen Magneten (13') umgeben, derart ausgerichtet sind, dass die Längsachsen der Magnete in der Anordnung (13") parallel zu der Längsachse des einzelnen Magneten (13') sind.

6. THR-Prothese nach Anspruch 3 oder 4, wobei in der Hüftgelenkpfannenkomponente (1) die Magnete in der Anordnung (13"), die den einzelnen Magneten (13') an der zentralen Wölbung (10) umgeben, derart ausgerichtet sind, dass die Längsachsen der Magnete in der Anordnung (13") zu der Längsachse des einzelnen Magneten (13') abgewinkelt sind.

7. THR-Prothese nach einem der Ansprüche 1-6, wobei sich in der Oberschenkelkomponente (21) das verjüngte Volumen (31) von der Außenfläche (29) des kugelförmigen Kopfs (28) nach innen erstreckt,
wobei das verjüngte Volumen (31) durch eine Endfläche (32), die die innerste Fläche des verjüngten Volumens (31) ist, und Wände (33), die sich von der Außenfläche (29) zu der Endfläche (32) erstrecken, definiert ist, und
wobei die Querschnittsfläche des verjüngten Volumens (31) von der Außenfläche (29) des kugelförmigen Kopfs (28) zu der Endfläche (32) hin abnimmt.

8. THR-Prothese nach Anspruch 7, wobei sich in der Oberschenkelkomponente (21) der eine oder die mehreren Magnete (34) an der Endfläche (32) des verjüngten Volumens (31), an den Wänden (33) des verjüngten Volumens (31), in die Dicke (30) des kugelförmigen Kopfs (28) angrenzend an die Endfläche (32) des verjüngten Volumens (31) eingebettet, in die Dicke (30) des kugelförmigen Kopfs (28) angrenzend an die Wände (33) des verjüngten Volumens (31) eingebettet oder eine Kombination davon befinden.

9. THR-Prothese nach Anspruch 7 oder 8, wobei in der Oberschenkelkomponente (21) der eine oder die mehreren Magnete (34) einen einzelnen Magneten (34') an der Endfläche (32), eine Anordnung von Magneten (34") an der Endfläche (32) oder sowohl einen einzelnen Magneten (34') an der Endfläche (32) als auch eine Anordnung von Magneten (34"), die den einzelnen Magneten (34') umgeben, umfassen.

10. THR-Prothese nach einem der Ansprüche 7-9, wobei in der Oberschenkelkomponente (21) der einzelne Magnet (34') an der Endfläche (32) so ausgerichtet ist, dass die Längsachse des einzelnen Magneten (34') senkrecht zu der Endfläche (32) ist, oder so ausgerichtet ist, dass die Längsachse des einzelnen Magneten (34') in der Oberschenkelkomponente (21) parallel zu dem einzelnen Magneten (13') in der Hüftgelenkpfannenkomponente (1) ist, wenn sich die Totalhüftersatz-Prothese in einer 0°-Hüftposition befindet.

11. THR-Prothese nach einem der Ansprüche 7-10, wobei in der Oberschenkelkomponente (21) der eine oder die mehreren Magnete (34) einen einzelnen Magneten (34') an der Endfläche (32) und eine Anordnung von Magneten (34"), die den einzelnen Magneten (34') umgeben, umfassen und die Magnete in der Anordnung (34"), die den einzelnen Magneten (34') umgeben, den gleichen Abstand zu dem einzelnen Magneten (34'), den gleichen Abstand zueinander oder eine Kombination davon aufweisen.

12. THR-Prothese nach Anspruch 11, wobei in der Oberschenkelkomponente (21) die Magnete in der Anordnung (34"), die den einzelnen Magneten (34') an der Endfläche (32) umgeben, derart ausgerichtet sind, dass die Längsachsen der Magnete in der Anordnung (34") parallel zu der Längsachse des einzelnen Magneten (34') sind.

13. THR-Prothese nach Anspruch 11, wobei in der Oberschenkelkomponente (21) die Magnete in der Anordnung (34"), die den einzelnen Magneten (34') an der Endfläche (32) umgeben, derart ausgerichtet sind, dass die Längsachsen der Magnete in der Anordnung (34") zu der Längsachse des einzelnen Magneten (34') abgewinkelt sind.

14. THR-Prothese nach einem der Ansprüche 1-13 zur Verwendung bei der Behandlung eines Subjekts, das einen THR benötigt.

15. Totalhüftersatz (THR)-Prothese, umfassend
(a) eine Hüftgelenkpfannenkomponente (1), die eine vollständige oder teilweise halbkugelförmige Form aufweist, die eine zentrale Wölbung (10), einen Umfang (12) und eine Zwischenwand (11) dazwischen umfasst, wobei die Hüftgelenkpfannenkomponente (1) eine Schale (2) und eine oder mehrere Bohrungen umfasst; wobei die eine oder die mehreren Bohrungen eine einzelne Bohrung an der zentralen Wölbung (10) und eine Anordnung von Bohrungen, die die einzelne Bohrung an der zentralen Wölbung (10) umgeben, umfassen, wobei die Bohrungen in der Anordnung den gleichen Abstand zu der einzelnen Bohrung an der zentralen Wölbung (10) aufweisen; und wobei die Schale (2) eine konkave Innenfläche (3), eine konvexe Außenfläche (5) und eine Dicke (4) dazwischen umfasst; und
(b) eine Oberschenkelkomponente (21), umfassend
(i) einen Schaftabschnitt (22), der ein proximales Ende (23) und ein distales Ende (24) umfasst,
(ii) einen Halsabschnitt (25), der ein verjüngtes Ende (26) und ein Basisende (27) umfasst, wobei das Basisende (27) des Halsabschnitts (25) mit dem proximalen Ende (23) des Schaftabschnitts (22) verbunden ist und sich der Halsabschnitt (25) in einem Winkel von dem Schaftabschnitt (22) erstreckt, und
(iii) einen kugelförmigen Kopf (28), der dazu konfiguriert ist, an dem verjüngten Ende (26) des Halsabschnitts (25) befestigt zu werden, wobei der kugelförmige Kopf (28) eine oder mehrere Bohrungen umfasst;
wobei der kugelförmige Kopf (28) ein verjüngtes Volumen (31), eine Außenfläche (29) und eine Dicke (30) zwischen dem verjüngten Volumen (31) und der Außenfläche (29) umfasst und wobei das verjüngte Volumen (31) dazu konfiguriert ist, das verjüngte Ende (26) des Halsabschnitts (25) aufzunehmen;
wobei die Hüftgelenkpfannenkomponente (1) dazu konfiguriert ist, den gesamten kugelförmigen Kopf (28) der Oberschenkelkomponente (21) oder einen Teil davon aufzunehmen; und
wobei jede der einen oder mehreren Bohrungen in der Schale der Hüftgelenkpfannenkomponente und jede der einen oder mehreren Bohrungen in dem kugelförmigen Kopf dazu konfiguriert sind, einen Magneten aufzunehmen.

## Revendications

1. Prothèse totale de hanche (PTH) comprenant
(a) un composant acétabulaire (1) de forme entièrement ou en partie hémisphérique qui comprend un dôme central (10), une périphérie (12) et une paroi intermédiaire (110) entre ceux-ci, où le composant acétabulaire (1) comprend une coque (2) et un ou plusieurs aimants (13) ; et où la coque (2) comprend une surface interne concave (3), une surface externe convexe (5) et une épaisseur (4) entre celles-ci ; et
(b) un composant fémoral (21) comprenant
(i) une portion formant tige (22) dotée d'une extrémité proximale (23) et d'une extrémité distale (24),
(ii) une portion formant col (25) dotée d'une extrémité effilée (26) et d'une extrémité basale (27), où l'extrémité basale (27) de la portion formant col (25) est jointe à l'extrémité proximale (23) de la portion formant tige (22) et où la portion formant col (25) se prolonge à un angle depuis la portion formant tige (22) et
(iii) une tête sphérique (28) qui est configurée pour être fixée à l'extrémité effilée (26) de la portion formant col (25), où la tête sphérique (28) comprend un ou plusieurs aimants (34) ;
où la tête sphérique (28) comprend un volume effilé (31), une surface extérieure (29) et une épaisseur (30) entre le volume effilé (31) et la surface extérieure (29) et où le volume effilé (31) est configuré pour recevoir l'extrémité effilée (26) de la portion formant col (25) ;
où le composant acétabulaire (1) est configuré pour recevoir la totalité ou une partie de la tête sphérique (28) du composant fémoral (21) ; et
où les un ou plusieurs aimants (13) du composant acétabulaire (1) et les un ou plusieurs aimants (34) de la tête sphérique 28 du composant fémoral 21 sont orientés de manière à générer une force d'attraction entre ceux-ci, **caractérisé en ce que** les un ou plusieurs aimants (13) comprennent un aimant unitaire (13') situé au niveau du dôme central (10) et un réseau d'aimants (13") entourant l'aimant unitaire (13') situé au niveau du dôme central (10), où les aimants (13") du réseau sont équidistants à l'aimant unitaire (13') situé au niveau du dôme central (10).

2. PTH de la revendication 1 où, dans le composant acétabulaire (1), l'axe long de l'aimant unitaire (13') situé au niveau du dôme central (10) est perpendiculaire à la ligne tangente de la courbure du dôme central (10).

3. PTH de la revendication 1 ou 2 où, dans le composant acétabulaire (1), le réseau d'aimants (13") entourant l'aimant unitaire (13') comprend de 2 à 16 aimants.

4. PTH de la revendication 1 où, dans le composant acétabulaire (1), les aimants du réseau (13") entourant l'aimant unitaire (13') situé au niveau du dôme central (10) sont équidistants les uns des autres.

5. PTH de la revendication 3 ou 4 où, dans le composant acétabulaire (1), les aimants du réseau (13") entourant l'aimant unitaire (13') sont orientés de manière que les axes longs des aimants du réseau (13") sont parallèles à l'axe long de l'aimant unitaire (13').

6. PTH de la revendication 3 ou 4 où, dans le composant acétabulaire (1), les aimants du réseau (13") entourant l'aimant unitaire (13') situé au niveau du dôme central (10) sont orientés de manière que les axes longs des aimants du réseau (13") sont à un angle par rapport à l'axe long de l'aimant unitaire (13").

7. PTH de l'une quelconque des revendications 1-6 où, dans le composant fémoral (21), le volume effilé (31) se prolonge vers l'intérieur depuis la surface extérieure (29) de la tête sphérique (28),
où le volume effilé (31) est défini par une surface terminale (32) qui est la surface la plus interne du volume effilé (31) et par des parois (33) qui se prolongent de la surface extérieure (29) à la surface terminale (32) et
où la superficie de la section du volume effilé (31) diminue vers la surface terminale (32) depuis la surface extérieure (29) de la tête sphérique (28).

8. PTH de la revendication 7 où, dans le composant fémoral (21), les un ou plusieurs aimants (34) sont situés au niveau de la surface terminale (32) du volume effilé (31), situés au niveau des parois (33) du volume effilé (31), encastrés dans l'épaisseur (30) de la tête sphérique (28) à un emplacement adjacent à la surface terminale (32) du volume effilé (31), encastrés dans l'épaisseur (30) de la tête sphérique (28) à un emplacement adjacent aux parois (33) du volume effilé (31) ou agencés selon une combinaison de ces options.

9. PTH de la revendication 7 ou 8 où, dans le composant fémoral (21), les un ou plusieurs aimants (34) comprennent un aimant unitaire (34') situé au niveau de la surface terminale (32), un réseau d'aimants (34") situés au niveau de la surface terminale (32) ou à la fois un aimant unitaire (34') situé au niveau de la surface terminale (32) et un réseau d'aimants (34") entourant l'aimant unitaire (34').

10. PTH de l'une quelconque des revendications 7-9 où, dans le composant fémoral (21), l'aimant unitaire (34') situé au niveau de la surface terminale (32) est orienté de manière que l'axe long de l'aimant unitaire (34') est perpendiculaire à la surface terminale (32) ou est orienté de manière que l'axe long de l'aimant unitaire (34') situé dans le composant fémoral (21) est parallèle à l'aimant unitaire (13') situé dans le composant acétabulaire (1) quand la prothèse totale de hanche est en position neutre (0°).

11. PTH de l'une quelconque des revendications 7-10 où, dans le composant fémoral (21), les un ou plusieurs aimants (34) comprennent un aimant unitaire (34') situé au niveau de la surface terminale (32) et un réseau d'aimants (34") entourant l'aimant unitaire (34') et où les aimants du réseau (34") entourant l'aimant unitaire (34') sont équidistants à l'aimant unitaire (34'), équidistants les uns des autres ou agencés selon une combinaison de ces options.

12. PTH de la revendication 11 où, dans le composant fémoral (21), les aimants du réseau (34") entourant l'aimant unitaire (34') situé au niveau de la surface terminale (32) sont orientés de manière que les axes longs des aimants du réseau (34") sont parallèles à l'axe long de l'aimant unitaire (34').

13. PTH de la revendication 11 où, dans le composant fémoral (21), les aimants du réseau (34") entourant l'aimant unitaire (34') situé au niveau de la surface terminale (32) sont orientés de manière que les axes longs des aimants du réseau (34") sont à un angle par rapport à l'axe long de l'aimant unitaire (34').

14. PTH de l'une quelconque des revendications 1-13 pour une utilisation dans le traitement d'un sujet qui requiert une PTH.

15. Prothèse totale de hanche (PTH) comprenant
(a) un composant acétabulaire (1) de forme entièrement ou en partie hémisphérique qui comprend un dôme central (10), une périphérie (12) et une paroi intermédiaire (11) entre ceux-ci, où le composant acétabulaire (1) comprend une coque (2) et un ou plusieurs alésages ; où les un ou plusieurs alésages comprennent un alésage unique aménagé au niveau du dôme central (10) et un réseau d'alésages entourant l'alésage unique aménagé au niveau du dôme central (10), où les alésages du réseau sont équidistants à l'alésage unique aménagé au niveau du dôme central (10) ; et où la coque (2) comprend une surface interne concave (3), une surface externe convexe (5) et une épaisseur (4) entre celles-ci ; et
(b) un composant fémoral (21) comprenant
(i) une portion formant tige (22) dotée d'une extrémité proximale (23) et d'une extrémité distale (24),
(ii) une portion formant col (25) dotée d'une extrémité effilée (26) et d'une extrémité basale (27), où l'extrémité basale (27) de la portion formant col (25) est j ointe à l'extrémité proximale (23) de la portion formant tige (22) et où la portion formant col (25) se prolonge à un angle depuis la portion formant tige (22) et
(iii) une tête sphérique (28) qui est configurée pour être fixée à l'extrémité effilée (26) de la portion formant col (25), où la tête sphérique (28) comprend un ou plusieurs alésages ;
où la tête sphérique (28) comprend un volume effilé (31), une surface extérieure (29) et une épaisseur (30) entre le volume effilé (31) et la surface extérieure (29) et où le volume effilé (31) est configuré pour recevoir l'extrémité effilée (26) de la portion formant col (25) ;
où le composant acétabulaire (1) est configuré pour recevoir la totalité ou une partie de la tête sphérique (28) du composant fémoral (21) ; et
où chacun des un ou plusieurs alésages aménagés dans la coque du composant acétabulaire et chacun des un ou plusieurs alésages aménagés dans la tête sphérique sont configurés pour recevoir un aimant.
